# EUROPÄISCHE PATENTSCHRIFT

(11) **EP 1 177 178 B1**
(45) Veröffentlichungstag und Bekanntmachung des Hinweises auf die Patenterteilung: **29.12.2004**
(21) Anmeldenummer: 00927088.5
(22) Anmeldetag: 27.04.2000
(51) Int. Cl.: C07D 235/18, C07D 235/20, C07D 401/04, C07D 403/04, C07D 417/04, C07D 413/04, C07D 409/04, C07D 471/04, C07D 401/14, C07D 405/04, A61K 31/415

(54) **HETEROZYKLISCH SUBSTITUIERTE BENZIMIDAZOLE, DEREN HERSTELLUNG UND ANWENDUNG**
HETEROCYCLICALLY SUBSTITUTED BENZIMIDAZOLES, THE PRODUCTION AND APPLICATION THEREOF
BENZIMIDAZOLES SUBSTITUES PAR VOIE HETEROCYCLIQUE, LEUR PRODUCTION ET LEUR APPLICATION

(30) Priorität: 07.05.1999 DE 19920936
(43) Veröffentlichungstag der Anmeldung: 06.02.2002
(73) Patentinhaber: Abbott GmbH & Co. KG, 65205 Wiesbaden (DE)
(72) Erfinder: LUBISCH, Wilfried, D-69115 Heidelberg (DE); KOCK, Michael, D-67105 Schifferstadt (DE); HÖGER, Thomas, D-68535 Edingen-Neckarhausen (DE); GRANDEL, Roland, D-69221 Dossenheim (DE); HOLZENKAMP, Uta, D-67245 Lambsheim (DE); SCHULT, Sabine, D-67346 Speyer (DE); MÜLLER, Reinhold, D-67105 Schifferstadt (DE)
(74) Vertreter: Grünecker, Kinkeldey, Stockmair & Schwanhäusser Anwaltssozietät
(86) Internationale Anmeldenummer: PCT/EP2000/003813
(87) Internationale Veröffentlichungsnummer: WO 2000/068206

(56) Entgegenhaltungen:
- EP-A- 0 305 008
- WO-A-00/26192
- WO-A-00/29384
- WO-A-00/32579
- WO-A-97/04771
- WO-A-97/48697
- ROGER J GRIFFIN ET AL: "Resistance modifying agents.3. Novel benzimidazole and quinnazolinone inhibitors of the DNA repair enzyme poly(ADP-ribose)polymerase" CHEMICAL ABSTRACTS + INDEXES,US,AMERICAN CHEMICAL SOCIETY. COLUMBUS, Bd. 15, Nr. 125, 12. Oktober 1998 (1998-10-12), Seite 521 XP002128895 ISSN: 0009-2258
- DENNY W A ET AL: "POTENTIAL ANTITUMOR AGENTS 59. STRUCTURE-ACTIVITY RELATIONSHIPS FOR 2-PHENYLBENZIMIDAZOLE-4-CARB OXAMIDES, A NEW CLASS OF MINIMAL DNA-INTERCALATING AGENTS WHICH MAY NOT ACT VIA TOPOISOMERASE II" JOURNAL OF MEDICINAL CHEMISTRY,US,AMERICAN CHEMICAL SOCIETY. WASHINGTON, Bd. 33, Nr. 2, 1. Februar 1990 (1990-02-01), Seiten 814-819, XP002007402 ISSN: 0022-2623 in der Anmeldung erwähnt

## Beschreibung

Die vorliegende Erfindung betrifft neuartige Benzimidazole, ihre Herstellung und die Verwendung als Inhibitoren des Enzyms Poly(ADP-ribose)polymerase oder PARP (EC 2.4.2.30) zur HerStellung von Arzneimitteln.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., *J. Histochem*. *Cytochem.* 1983, *31*, 1261-1264). Man nimmt an, daß PARP eine Rolle bei der Reparatur von DNA-Brüchen spielt (M.S. Satoh et al., *Nature* 1992, *356*, 356-358). Schädigungen oder Brüche der DNA-Stränge aktivieren das Enzym PARP, das, wenn es aktiviert ist, die Übertragung von ADP-Ribose aus NAD katalysiert (S. Shaw, *Adv. Radiat. Biol.,* 1984, *11*, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw Organschäden führen oder beitragen. Dazu zählen zum Beispiel ischämische Zustände von Organen wie im Schlaganfall, Herzinfarkt (C. Thiemermann et al.. *Proc. Natl. Acad. Sci. USA,* 1997, *94*, 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von einer Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. *Cancer Chemo. Pharmacol.* 1988, *22,* 303). Nicht limitierende Beispiele für Tumoren sind Leukämie, Glioblastome, Lymphome, Melanome, Mama und Cervicalkarzinome.

Zudem wurde gefunden, daß PARP-Inhibitoren immunosuppressive Wirkung zeigen können (D. Weltin et al. *Int. J. Immunopharmacol.* 1995, *17*, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Infammation* 1996, *20*, 203-215; W. Ehrlich et al. *Rheumatol. Int.* 1995, *15*, 171-172; C. Szabo et al., *Proc. Natl. Acad. Sci*. *USA* 1998, *95*, 3867-3872; S. Cuzzocrea et al. *Eur. J. Pharmacol.* 1998, *342*, 67-76). Unter PARP im Sinne dieser Erfindung werden auch Isoenzyme des oben beschriebenen PARP-Enzyms verstanden.

Weiterhin zeigte der PARP-Inhibitor 3-Aminobenzamid protektive Effekte in einem Modell für den Kreislaufschock (S. Cuzzocrea et al., *Br. J. Pharmacol.* 1997, *121*, 1065-1074).

Ebenfalls gibt es experimentelle Hinweise, dass Inhibitoren des Enzymes PARP als Mittel zur Behandlung von Diabetes mellitus nützlich sein könnten (V. Burkart et al. *Nature Med.* 1999, *5*, 314-319).

Benzimidazole sind vielfach beschrieben worden. So sind in DE 38 30 060 alkylierte Derivate als Inhibitoren der Erythrozytenaggregation offengelegt. In DE 35 22 230 ist ein Ester-Derivat vom 2-Phenylbenzimidazol als Inhibitor der Plättchenaggregation aufgeführt. Halogen-substituierte 2-Phenylbenzimidazole, die am Phenyl-Ring substituierte Amin-Reste tragen, sind in WO 98/06703 als MCP-1-Antagonisten beschrieben worden.

Ebenfalls sind 2-Phenyl-benzimidazole bekannt, bei denen die Benzimidazol-Gruppe durch eine Amid-Gruppe substituiert ist. 5-Amido-Derivate des 2-Phenylbenzimidazols, die am Phenyl-Ring Alkyloxy-Reste tragen, sind in WO 94/12461 als Inhibitoren der cAMP-Phosphodiesterase beschrieben worden. Für analoge Derivate wurde in DE 35 46 575 (z.B. Beispiel 15) gefunden, daß diese Verbindungen positiv inotrope Effekte auslösen. Ebenfalls 4-Amido-Derivate, die in 3-Stellung ein Pyridyl-Rest tragen, sind in WO 97/48697 als Inhibitoren der cAMP-Phosphodiesterase aufgeführt.

Benzimidazole, die in 4-Stellung Amido-Gruppen tragen, mit heterocyclischen Ringen in 2-Stellung sind ebenfalls bekannt, zum Beispiel in Denn W.A. et al., J. Med. Chem. 1990, 33, 814-819. Dort sind zum Beispiel Benzimidazole mit Thiophen-Ringen, mit Pyridin-Ringe, Furan-Ringe und Pyrrol-Ringen in 2-Stellung beschrieben, allerdings tragen die Amido-Gruppen in 4-Stellung am Benzimidazol weitere Alkyl-Amino-Reste, die wichtig für die dort erwähnte zytotoxische Wirkung ist, für eine inhibitorische Wirkung gegenüber dem Enzym PARP sind diese Substitutionen am Amid-Rest jedoch äußerst ungünstig und führen in der Regel zu inaktiven Verbindungen (s.S. 728 in M.J. Suto et al., Drugs of the Future, 1991, 16, 723-739)

Die Synthese von 2-Phenyl-benzimidazyl-4-amiden ist in J. Chem. Soc. Perkin Trans 1, 1979, 2303-2307 beschrieben worden. Analoge Verbindungen, die am Amid-Rest noch eine substituierte AlkylKette tragen, und die cytotoxische Wirkung haben sollen, sind in J. Med. Chem. 1990, 33, 814-819 aufgeführt. In WO 97/04771 sind dagegen Benzimidazol-4-amide aufgeführt, die das PARS hemmen. Insbesondere sind Derivate dort als wirksam beschrieben , die einen Phenyl-Ring in 2-Stellung tragen, wobei der Phenyl-Ring noch mit einfachen Substituenten wie Nitro, Methoxy und CF₃, substituiert sein kann. Obwohl diese Substanzen zum Teil gute Hemmung des Enzyms PARP zeigen, haben die dort beschriebenen Derivate als Nachteil, daß sie nur wenig oder keine Löslichkeit in wäßrigen Lösungen zeigen und somit nicht als wäßrige Lösung appliziert werden können.

In einer Reihe von Therapien wie Schlaganfall werden die Wirkstoffe intravenös als Infusionslösung appliziert. Dazu ist es notwendig, Substanzen, hier PARP-Inhibitoren, zur Verfügung zu haben, die ausreichende Wasserlöslichkeit bei physiologischen pH-Werten oder angenäherten pH-Werten (z.B: pH-Werten von 5-8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. WO 97/04771 ). Diese Hilfsstoffe, zum Beispiel Polyethylenglykol und Dimethylsulfoxid, verursachen häufig Nebeneffekte oder sind sogar unverträglich. Gut wirksame PARP-Inhibitoren mit ausreichender Wasserlöslichkeit sind bisher nicht beschrieben worden.

Benzimidazole, die in 5-Stellung eine Carbonsäureester- bzw. eine Carbonsäureamidgruppe tragen und gleichzeitig in 2-Stellung heteroaromatische Ringe tragen, sind selten beschrieben worden, so z.B. Thiazole (JP 4001631) und Chinoline (WO 9820007). Benzimidazole, die am Benzo-Ring zum Beispiel Methyl-Gruppen tragen oder am Benzo-Ring weitere anellierte Benz-Ringe haben oder sogar unsubstituiert sind, sind schon öfter mit heteroaromatischen Ringen in 2-Stellung beschrieben worden, so zum Beispiel Indole (V. Ketarev et al., Chem. Heterocycl. Comp. 1980, 16, 501-506), Chinoline (J. Gosh, J. Ind. Chem. Soc. 1938, 15, 89), Pyridine (T. Hisano, Chem. Pharm. Bull 1982, 30, 2996-3004), Pyrimidine (H. Bredereck et al., Chem. Ber. 1960, 93, 2410-2414) und Pyrrole (GB 966,796).

Benzimidazole mit hetreoaromatischen Ringen wie Pyridin, Furan Thiophen und Pyrrol in 2-Stellung, die in 4-Stellung Karbonsäure-Derivate tragen wurden in W.A. Denny et al., J. Med. Chem. 1990, 33, 814-819 als potentielle Zytostatika beschrieben. Dabei sind aber als Karbonsäure-Derivate nur die Karbonsäure selbst und Amide, die am N-Atom noch Alkylamin-Reste tragen, hergestellt und erwähnt worden.

Es wurde überraschenderweise gefunden, daß Benzimidazole, die am Imidazol-Ring auch heteroaromatische Ringe und die in 4-Stellung eine primäre Karbonsäureamid-Gruppe tragen, also im Gegensatz zu W.A: Denny et al (s. oben) keine weiteren Reste am Säureamid-N-Atom tragen, gut wirksame Inhibitoren für das Enzym PARP darstellen. Durch den weiteren Einbau von chemischen Resten wie aliphatischen Aminen kann durch eine Salzbildung, zum Beispiel mit Säuren, zusätzlich eine deutlich verbesserte Wasserlöslichkeit erreicht werden.

In der vorliegenden Erfindung werden neue Benzimidazol-Derivate der allgemeinen Formeln I und II beschrieben, die gegenüber den bereits beschriebenen Verbindungen Vorteile zeigen und potente PARP-Inhibitoren darstellen und zum Teil auch ausreichende Wasserlöslichkeit zeigen, die eine Applikation als Infusionslösung ermöglicht.

Gegenstand der vorliegenden Erfindung sind substituierte Benzimidazole der allgemeinen Formeln I und II: 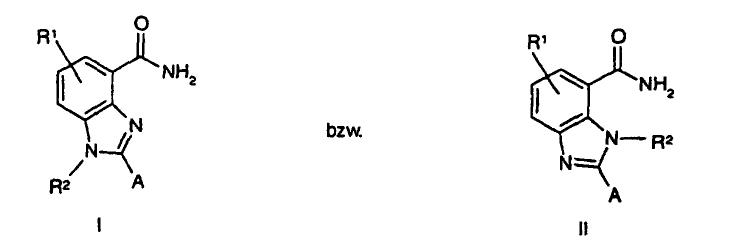 worin
- A: aromatischer Heteromonocyclus, aromatischer oder teilaromatischer Heterobicyclus und Heterotricyclus bedeutet, wobei die Ringsysteme maximal 15 Kohlenstoffatome und bis zu 4 Heteroatome ausgewählt aus der Gruppe N,O,S enthalten und Cyclen zusätzlich noch bis zu 2 Oxogruppen tragen können und A noch mit bis zu drei unterschiedlichen oder gleichen Resten R³ und zusätzlich einem Rest R⁴ substituiert sein kann und
- R¹: Wasserstoff, Chlor, Fluor, Brom, Jod, verzweigtes und unverzweigtes C₁-C₆-Alkyl, OH, Nitro, CF₃, CN, NR¹¹R¹², NH-CO-R¹³, O-C₁-C₄-Alkyl, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeuten, und
- R²: Wasserstoff, verzweigtes und unverzweigtes C₁-C₆-Alkyl und
- R³: Wasserstoff, Chlor, Brom, Iod, Fluor, CF₃, OCF₃, Nitro, NH₂, CO-R⁸, CO₂-R⁸, SO₂-R⁸, OH, O-C₁-C₄-Alkyl, O-C₀-C₄-Alkyl-Phenyl, eine C₁-C₆-Kette, die gesättigt, ungesättigt oder partiell ungesättigt sein kann, und noch mit einem Rest R³³ substituiert sein kann, Phenyl, wobei die Phenyl-Ringe noch mit bis zu drei gleichen oder verschiedenen Resten R³¹ subsitutiert sein können, und Pyridyl, den mit bis zu drei Resten R³² substituiert sein kann, und
- R³¹: OH, C₁-C₆-Alkyl, O-C₁-C₄-Alkyl, Chlor, Brom, Jod, Fluor, CF₃, Nitro, NH₂, und
- R³²: OH, C₁-C₆-Alkyl, O-C₁-C₄-Alkyl, Chlor, Brom, Iod, Fluor, CF₃, Nitro, NH₂, CN, und
- R³³: CO-NH-R⁸, OH, O-C₁-C₆-Alkyl. O-CO-R⁸, und
- R⁴: -(D)ₚ-(E)ₛ-(CH₂)_{q} -B bedeutet, wobei
- D: S, N-R⁴³ und O
- E: Phenyl und
- s: 0 und 1 und
- B: NR⁴¹R⁴² und 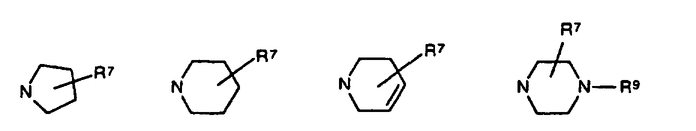 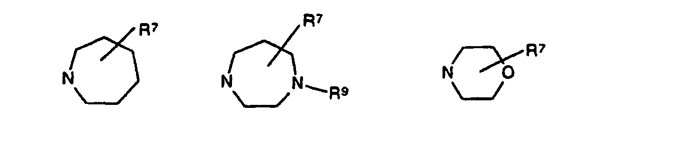
bedeutet, wobei
- p: 0 und 1 bedeuten kann und
- q: 0, 1, 2, 3 oder 4 sein kann, und
- R⁴¹: Wasserstoff, C₁-C₆-Alkyl, (CH₂)ᵣ-G und
- R⁴²: Wasserstoff, C₁-C₆-Alkyl, -CO-R⁸, SO₂-R⁸, CO₂-R⁸, -(C=N)-R⁸ und -(C=N)-NHR⁸ und
- R⁴¹ und R⁴²: einen Phthaloyl-Rest bilden können und
- R⁴³: Wasserstoff und C₁-C₄-Alkyl und
- r: 0,1,2,3,4 und
- G: Phenyl, der noch maximal zwei Reste R tragen kann, NR¹¹R¹², NH-C₁-C₄-Alkyl-Phenyl, Pyrrolidin, Piperidin, 1,2,5,6-Tetrahydropyridin, Morpholin, Homopiperidin, Piperazin, das noch mit einem Alkyl-Rest C₁-C₆-Alkyl substituiert sein kann, und Homopiperazin, das noch mit einem Alkyl-Rest C₁-C₆-Alkyl substituiert sein kann, und
- R⁷: Wasserstoff, C₁-C₆-Alkyl, Phenyl, wobei der Ring noch mit bis zu zwei Resten R⁷¹ substituiert sein kann, und
- R⁷¹: OH, C₁-C₆-Alkyl, O-C₁-C₄-Alkyl, Chlor, Brom. Jod, Fluor, CF₃, Nitro, NH₂, und
- R⁸: C₁-C₆-Alkyl, CF₃, NR¹¹R¹², Phenyl. C₁-C₄-Alkyl-Phenyl, wobei der Ring noch mit bis zu zwei Resten R⁸¹ substituiert sein kann, und
- R⁸¹: OH, C₁-C₆-Alkyl, O-C₁-C₄-Alkyl, Chlor, Brom, Jod, Fluor, CF₃, Nitro, NH₂, und
- R⁹: Wasserstoff, CO-R⁸, SO₂-R⁸, CO₂-R⁸, C₁-C₆-Alkyl, C₁-C₄-Alkyl-Phenyl und Phenyl, wobei die Phenyl-Ring- noch mit bis zu zwei Resten R⁹¹ substituiert sein kann, und
- R⁹¹: OH, C₁-C₆-Alkyl, O-C₁-C₄-Alkyl, Chlor, Brom, Jod, Fluor, CF₃, Nitro, NH₂, und sein kann,
sowie ihre tautomeren Formen, möglichen enantiomeren und diastereomeren Formen, und deren Prodrugs, ausgewählt aus der Gruppe bestehend aus Phosphaten, Carbamaten von Aminosäuren und Estern.

Bevorzugt werden die Verbindungen der Formel I und II, wobei
R¹ Wasserstoff und
R² Wasserstoff und C₁-C₄-Alkyl und
D NR⁴³ und O und
p 0 und 1 und s 0 und q 0, 1 und 2, wenn p = 0 ist, oder q 2 und 3, wenn p = 1 ist, und
R⁴² und R⁴³, unabhängig voneinander, Wasserstoff und C₁-C₄-Alkyl und
R⁷ Wasserstoff und Phenyl und
R⁹ Wasserstoff, C₁-C₄-Alkyl und C₀-C₄-Alkyl-Phenyl sein kann.

Bevorzugte Bedeutung von A sind Indol, Benzimidazol, Pyrrol, Imidazol. Furan, Thiophen, Benzothiophen, Benzofuran, Pyrazol, Thiazol. Benzothiazol, Phthalimid, Indazol, Benzotriazol, Phthalizin Indolin, Isoindolin, Pyridin, Chinolin, Pyrimidin, Pyridazin, Isochinolin, Chinoxalin, Chinazolin, Isooxazol, Oxazol, Imidazopyridin, Pyrazin.

Bevorzugt sind Verbindungen der Formel I und II, wobei A folgende Bedeutung hat:
Pyridin, Thiophen, Thiazol, Furan, Indol, Oxazol, Pyrazol, Pyrrol, Benzofuran, Imidazol, Benzothiophen, Isoxazol, Pyrazin, Pyrimidin, Pyridazin, Chinolin. und der Heterocyclus mit bis zu drei Resten R³ und einen Rest R⁴ substituiert sein kann, wobei
- R³: Wasserstoff, Chlor, Brom, Iod, Fluor, COR⁸, CO₂R⁸, SO₂R₈, eine C₁-C₆-Kette, die gesättigt, ungesättigt oder partiell ungesättigt sein kann, und noch mit einer Gruppe O-CO-R⁸ substituiert sein kann, C₁-C₆-Alkyl-Phenyl, Phenyl, wobei die Phenyl-Ringe noch mit bis zu drei gleichen oder verschiedenen Resten R³¹ substituiert sein können, und Pyridyl, das mit bis zu drei Resten R³² substituiert sein kann, und
- R⁴: Wasserstoff und (D)ₚ-(E)ₛ-(CH₂)_{q}-B, und R³ und R⁴ nicht gleichzeitig Wasserstoff sind.

Bevorzugt sind Verbindungen nach Formel I und II, wobei A folgende Bedeutung hat:
Pyridin, Pyrazin, Pyrimidin, Pyridazin, Chinolin, Thiazol, Thiophen, Pyrrol und Pyrazol und der Heterocyclus mit einem Rest R³ und einem Rest R⁴ substituiert sein kann, wobei
- R³: Wasserstoff, Chlor, Brom, Iod, Fluor, C₁-C₄-Alkyl, und
- R⁴: (D)ₚ-(E)ₛ-(CH₂)_{g}-B ist.

Besonders bevorzugt sind Verbindungen nach Formel I und II, wobei A Pyridin, Thiophen und Thiazol sein kann und der Heterocyclus mit einem Rest R⁴ stubstituiert ist, wobei R⁴ (D)ₚ-(E)ₛ-(CH₂)_{q}-B ist, und R³ Wasserstoff bedeutet.

Die Verbindungen der Formel I und II können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I und II oder ihren Zwischenprodukten durchführt.

Gegenstand der Erfindung sind auch zu Verbindungen der Formel I bzw. II mesomere oder tautomere Verbindungen.

Ein weiterer Gegenstand der Erfindung sind die physiologisch verträglichen Salze der Verbindungen I und II, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die in vivo in Verbindungen der allgemeinen Formel I und II metabolisiert werden.

Die Herstellung der erfindungsgemäßen Benzimidazole I und II kann auf verschiedenen Wegen erfolgen und wurde in den Syntheseschemata 1-3 skizziert.

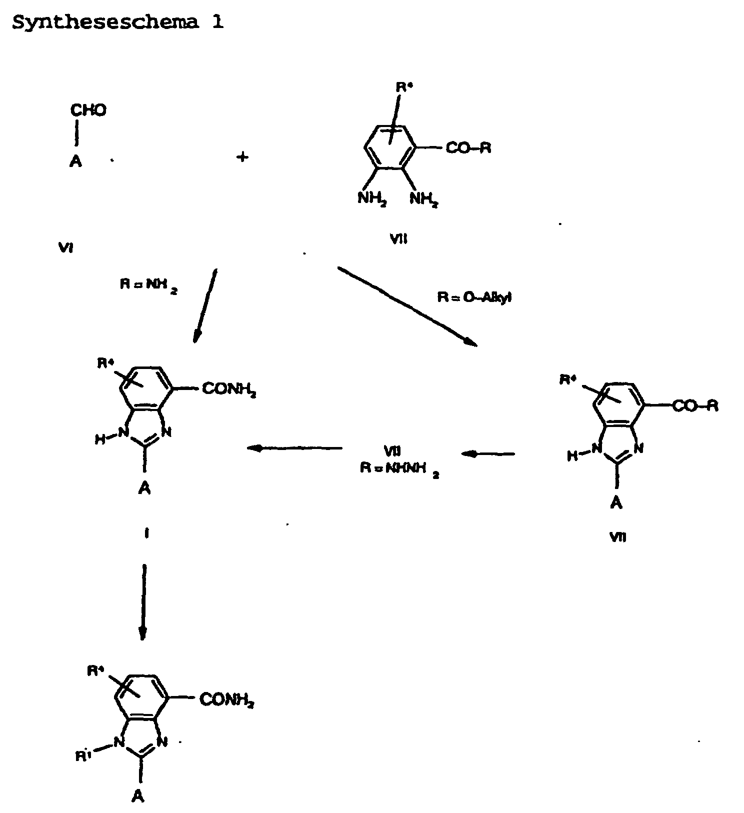

Durch Kondensation des Benzaldehyds mit Phenylendiaminen erhält man das Benzimidazol VII, wobei man bevorzugt in polaren Lösungsmitteln wie Ethanol oder Dimethylformamid und Zusatz von Säuren wie Essigsäure bei erhöhter Temperatur arbeitet, in der Regel 80 bis 120°C. Günstig für die Reaktion ist der Zusatz von schwachen Oxidationsmittel wie Kupfer-II-Salzen, die als wäßrige Lösung zugesetzt werden.

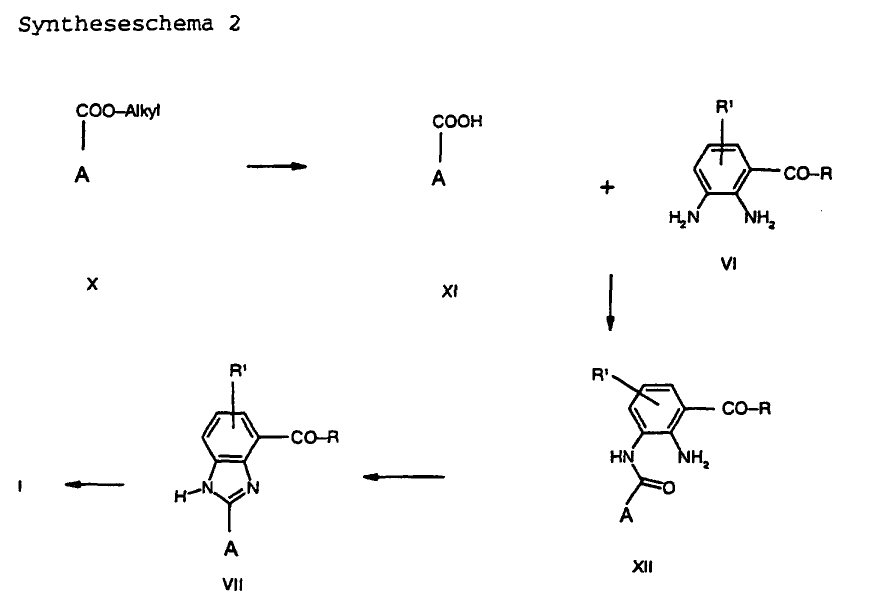

Wenn in dem Phenylendiamin VIII R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzten. Alternativ kann man den Ester VIII mit Hydrazin in polaren Lösungsmitteln wie die Alkohole Butanol und Ethanol oder auch Dimethylformamid, bei erhöhten Temperaturen, vorzugsweise 80-130°C, umsetzten, wobei ein Hydrazid VIII (R = NHNH₂) anfällt, das danach noch unter reduktiven Bedingungen, wie mit Raney-Nickel in Alkoholen unter Rückfluß, zum Amid I reduziert werden kann.

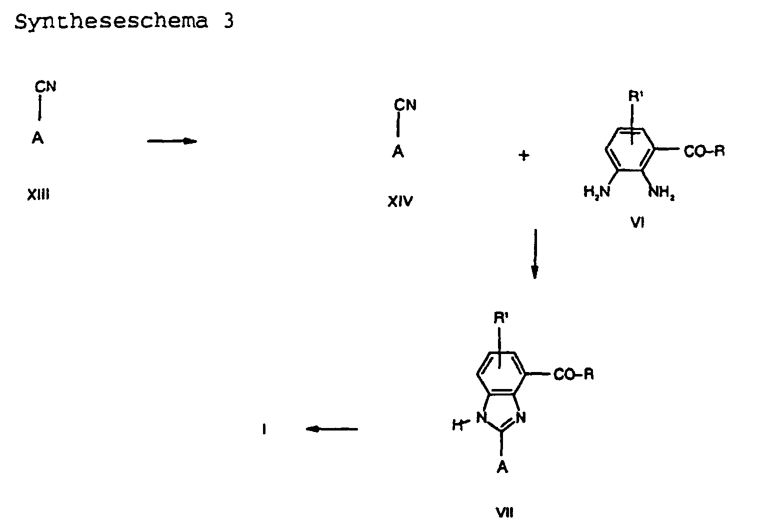

Alternativ zu den im Schema 1 gezeigten Benzaldehyden VI kann man auch Benzoesäuren wie XI (siehe Schema 2) oder Benzonitrile wie XIV(siehe Schema 3) anstelle des Benzaldehyds einsetzen. Die Herstellung dieser Derivate erfolgt analog zur Herstellung der substituierten Benzaldehyde VI. Ausgehend von XI erfolgt die Kondensation zu VII in zwei Stufen . Zuerst wird die Benzoesäure XI mit dem Anilin VI in einer peptidartigen Kupplung zum Amid XII umgesetzt. Dabei arbeitet man nach üblichen Bedingungen, die zum Beispiel im Houben-Weyl, Methoden der Organischen Chemie, 4. Aufl., E5, Kap. V bzw. C.R. Larock, Comprehensive Organic Transformations, VCH Publisher, 1989, Seite 972f. aufgelistet sind. Der Ringschluß erfolgt zum Benzimidazol erfolgt danach bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

Die Reaktion des Phenylendiamins VI mit einem Benzonitril XIV erfolgt ebenfalls unter üblichen Bedingungen. Dabei kann man in Lösungsmitteln wie Dimethylformamid unter Zusatz von Säuren oder auch in Polyphosphorsäure bei erhöhter Temperatur wie 60 bis 200°C arbeiten. Allerdings kann man auch die üblichen Methoden zur Herstellung von Amidinen aus Benzonitrilen anwenden, wie sie in Houben-Weyl, Methoden der organischen Chemie, E5, S. 1304 f., J. Amer. Chem. Soc. 1957, 427 und J. Org. Chem. 1987, 1017 beschrieben sind.

Die oben genannten substituierten Benzimidazole I und II stellen Inhibitoren des Enzyms Poly(ADP-ribose)polymerase oder PARP (EC 2.4.2.30) dar.

Die inhibitorische Wirkung der substituierten Benzimidazole I und II kann mit einem in der Literatur bereits bekannten Enzymtest ermittelt werden, wobei als Wirkmaßstab ein Ki-Wert ermittelt wird. Die Benzimidazole I und II wurden in dieser Weise auf Hemmwirkung des Enzyms Poly(ADP-ribose)polymerase oder PARP (EC 2.4.2.30) gemessen.

Die substituierten Benzimidazole der allgemeinen Formeln I und II stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Neben dem Enzym PARP sind auch PARP-Isoenzyme bekannt, wie z.B. PARP II und PARP III (WO 99/64572).

Überraschenderweise zeigten die Benzimidazole I und II auch Hemmwirkung des Enzyms PARP II.

Im Hinblick auf höhere Verträglichkeit und geringere Nebenwirkungen von Arzneimitteln ist eine selektive Hemmung der PARP-Enzyme wünschenswert.

Während das in WO 97/04771 beschriebene 2-Phenylbenzimidazol-4-carboxamid (NU 1070) die Enzyme PARP und PARP II mit Ki-Werten der gleichen Größenordnung hemmt, zeigen die Benzimidazole I und II verbesserte Selektivitäten in der Hemmung von PARP und PARP II.

Die Verbindungen der Formeln I und II können zur Herstellung von Arzneimitteln zur Behandlung von Schädigungen nach Ischämien und zur Prophylaxe bei erwarteten Ischämien verschiedener Organe eingesetzt werden.

Die vorliegenden Benzimidazole der allgemeinen Formel I und II können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. weiterhin können die Verbindungen der allgemeinen Formeln I und II zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden Benzimidazole I bzw. II zur Behandlung einer Revascularisation kritisch verengter Koronaraterien, zum Beispiel bei der PCTA und Bypass-Operationen, und kritisch verengter peripherer Arterien, zum Beispiel Beinarterien, dienen. Zudem können die Benzimidazole I bzw. II bei der Chemotherapie von Tumoren und deren Metastasierung nützlich sein und zur Behandlung von Entzündungen und rheumatischen Erkrankungen, wie z.B. rheumatischer Arthritis und auch zur Behandlung von Diabetes mellitus dienen.

Die erfindungsgemäßen Arzneimittelzubereitungen enthalten neben den üblichen Arzneimittel-hilfsstoffen eine therapeutisch wirksame Menge der Verbindungen I und II.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitungen können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel A: Hemmung des Enzyms Poly(ADP-ribose)polymerase oder PARP (EC 2.4.2.30)

a) ELISA-Assay
   Materialien:
   ELISA Farbreagenz: TMB-Fertigmix SIGMA T-8540
   Eine 96-well Mikrotiterplatte (FALCON Micro-Test IIIä Flexible Assay Plate, # 3912) wurde mit Histonen (SIGMA, H-7755) beschichtet. Histone wurden hierfür in Carbonatpuffer (0,05 M Na₂HCO₃; pH 9,4) in einer Konzentration von 50 µg/ml gelöst. Die einzelnen Wells der Mikrotiterplatte wurden mindestens 2 Stunden bei Raumtemperatur oder über Nacht bei 4°C mit je 150 µl dieser Histon-Lösung inkubiert. Anschließend werden die Wells durch Zugabe von 150 µl einer 1%igen BSA-Lösung (SIGMA, A-7888) in Carbonatpuffer für 2 Stunden bei Raumtemperatur blockiert. Es folgen drei Waschschritte mit Waschpuffer (0,05 % Tween10 in 1x PBS; PBS (Phosphate buffered saline; Gibco, Best.-Nr. 10010): 0,21 g/l KH₂PO₄, 9 g/l NaCl, 0,726 g/l Na₂HPO₄ · 7H₂O, pH 7,4). Waschschritte wurden durchweg mit einem Mikrotiterplatten-Waschgerät durchgeführt (Mikrotiterplatten-Wäscher "Columbus", SLT-Labinstruments, Österreich).
   Für die Enzymreaktion wurden eine Enzymreaktionslösung und eine Substratlösung jeweils als "Pre-Mix" benötigt. Die absolute Menge dieser Lösungen richtete sich nach der Anzahl der vorgesehenen Test-Wells.
   Zusammensetzung der Enzymreaktionslösung pro Well:
   - 4 µl PARP-Reaktionspuffer (1 M Tris-HCl pH 8,0, 100 mM MgCl₂, 10 mM DTT)
   - 20 ng PARP (human oder bovin)
   - 4 µl aktivierte DNA (1 mg/ml; SIGMA, D-4522)
   - ad 40 µl H₂O

   Zusammensetzung der Substrat-Lösung pro Well:
   - 5 µl PARP-Reaktionspuffer (10x)
   - 0,8 µl NAD-Lösung (10 mM, SIGMA N-1511)
   - 44 µl H₂O

   Inhibitoren wurden in 1x PARP-Reaktionspuffer gelöst. DMSO, das gelegentlich zum Lösen von Inhibitoren in höheren Konzentrationen verwendet wurde, war bis zu einer Endkonzentration von 2 % unproblematisch. Für die Enzymreaktion wurden 40 µl der Enzymreaktionslösung pro Well vorgelegt und mit 10 µl Inhibitor-Lösung für 10 Minuten inkubiert. Anschließend wurde die Enzymreaktion durch Zugabe von 50 µl Substrat-Lösung pro Well gestartet. Die Reaktion wurde 30 Minuten bei Raumtemperatur durchgeführt und anschließend durch dreimaliges Waschen mit Waschpuffer gestoppt.
   Als primäre Antikörper wurden spezifische Anti-Poly-(ADPribose) Antikörper in einer 1:5000 Verdünnung eingesetzt. Die Verdünnung erfolgte in Antikörper-Puffer (1 % BSA in PBS; 0,05 % Tween20). Die Inkubationszeit für den primären Antikörper betrug eine Stunde bei Raumtemperatur. Nach anschließendem dreimaligem Waschen mit Waschpuffer erfolgte eine einstündige Inkubation bei Raumtemperatur mit dem sekundärem Antikörper (Anti-Maus-IgG, Fab-Fragmente, Peroxidase gekoppelt, Boehringer Mannheim, Best.-Nr. 1500.686; Anti-Rabbit-IgG, Peroxidase gekoppelt, SIGMA, Best.-Nr. A-6154) in einer 1:10000 Verdünnung in Antikörperpuffer. Nach dreimaligem Waschen mit Waschpuffer folgte die Farbreaktion unter Verwendung von 100 µl Farbreagenz (TMB-Fertigmix, SIGMA) pro Well für ca. 15 min. bei Raumtemperatur. Die Farbreaktion wurde durch Zugabe von 100 µl 2M H₂SO₄ gestoppt. Danach wurde sofort im ELISA-Platten-Lesegerät ("Easy Reader" EAR340AT, SLT-Labinstruments, Österreich) gemessen (450 nm gegen 620 nm) .
   Für die Ermittlung des Kᵢ-Wertes eines Inhibitors wurden verschiedene Konzentrationen zur Erstellung einer Dosis-Wirkungskurve herangezogen. Für eine bestimmte Inhibitorkonzentration werden 3fach-Werte erhoben. Arithmetische Mittelwerte werden mit Microsoft© Excel ermittelt. Die IC₅₀-Bestimmung erfolgt mit der Microcal© Origin Software (Vers. 5.0) ("Sigmoidal Fit"). Umrechnung der so berechneten IC₅₀-Werte auf Kᵢ-Werte erfolgte durch Verwendung von "Eich-Inhibitoren". Die "Eich-Inhibitoren" wurden bei jeder Analyse mitgemessen. Der Kᵢ-Werte der "Eich-Inhibitoren" wurde im gleichen Testsystem durch Dixon-Diagramm Analyse in der dem Fachmann geläufigen Weise ermittelt.
b) HTRF-(Homogenous time-resolved fluorescence) Assay
   Beim HTFR-PARP-Assay werden Histone als Zielproteine der Modifikation durch PARP indirekt mit einem XL665-Fluorophor markiert. Der Antikörper wird direkt mit einem Europium-Kryptat markiert. Befindet sich das XL665-Fluorophor in einer unmittelbaren räumlichen Nähe, die durch eine Bindung an die Poly-(ADP-ribose) am Histon gewährleistet wird, dann ist eine Energieübertragung möglich. Die Emission bei 665 nm ist somit direkt proportional zu der Menge an gebundenem Antikörper, der wiederum der Poly-(ADP-ribose) Menge entspricht. Somit entspricht das gemessene Signal der PARP Aktivität. Die verwendeten Materialien sind, wenn nicht ausdrücklich angegeben, identisch mit denen im ELISA Assay (s.o.) verwendeten.
   Histone (Sigma M7755) wurden in Hepes-Puffer (50 mM, pH = 7,5) zu 3 mg/ml gelöst. Biotinylierung erfolgte mit Sulfo-NHS-LC-Biotin (Pierce, # 21335T). Ein molares Verhältnis von 4 Biotin pro Histon wurde verwendet. Die Inkubationszeit betrug 90 Minuten (RT). Anschließend wurden die biotinylierten Histone über eine G25 SF HR10/10 Säule (Pharmacia, 17-0591-01) in Hepes Puffer (50 mM, pH = 7,0) aufgereinigt, um überschüssiges Biotinylierungsreagenz zu entfernen. Der Anti-Poly-(ADP-ribose)-Antikörper wurde mittels bifunktionaler Kopplungsreagenzien mit Europium-Kryptat markiert. (Lopez E. et al. Clin. Chem. 39/2, 196-201, 1993 US P 5,534,662) Die Reinigung erfolgte auf einer G25SF HR10/30 Säule. Ein molares Verhältnis von 3,1 Kryptaten pro Antikörper wurde erzielt. Die Ausbeute betrug 25 %.
   Die Konjugate wurden in Gegenwart von 0,1 % BSA in Phosphatpuffer (0,1 M, pH = 7) bei -80°C gelagert.
   Für die Enzymreaktion wurden pro Well zusammenpipettiert:
   - 10 µl PARP-Lösung in PARP-HTRF-Reaktionspuffer (50 mM Tris-HCl pH 8,0, 10 mM MgC12, 1 mM DTT) mit 20 ng PARP (human oder bovin)
   - 10 µl aktivierte DNA (SIGMA D4522) in PARP-HTRF-Reaktionspuffer (50 µg/ml)
   - 10 µl biotinylierte Histone in PARP-HTRF-Reaktionspuffer (1,25 µM)
   - 10 µl Inhibitor in PARP-HTRF-Reaktionspuffer

   Diese Reagenzien wurden 2 Minuten vorinkubiert, bevor die Reaktion durch Zugabe von
   - 10 µl NAD-Lösung in PARP-HTRF-Reaktionspuffer (400 µM) gestartet wurde. Die Reaktionszeit betrug 30 Minuten bei Raumtemperatur.

   Anschließend wurde die Reaktion durch Zugabe von
   - 10 µl PARP-Inhibitor (25 µM, Kᵢ = 10 nM) in "Revelation"-Puffer (100 mM Tris-HCl pH 7,2, 0,2 M KF, 0,05 % BSA)
   gestoppt.
   Danach wurden zugegeben:
   - 10 µl EDTA-Lösung (SIGMA, E-7889, 0,5 M in H₂O)
   - 100 µl Sa-XL665 (Packard Instruments) in "Revelation"-Puffer (15-31,25 nM)
   - 50 µl Anti-PAR-Kryptat in "Revelation"-Puffer (1,6-3,3 nM).

   Nach 30 Minuten (bis 4 Stunden) konnte dann gemessen werden. Die Messung erfolgte auf einem "Discovery HTRF Microplate Analyzer" (Packard Instruments). Die Berechnung der Ki-Werte erfolgte wie beim ELISA Assay beschrieben.

### Beispiel B: Bestimmung des Wasserlöslichkeit

Eine zu messende Verbindung wird direkt in einem festgelegten Volumen Wasser gelöst und die entstandene Lösung mit einer Natriumacetat-Lösung auf pH-5-6 eingestellt, so daß die zu prüfende Konzentration des Wirkstoffs erreicht wird. Fall die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid ≤ 1 %), wonach auch hier der pH-Wert noch eingestellt wurde. Der potente PARP-Inhibitor NU 1076 (WO 97/04771) zeigte hier eine Löslichkeit < 0,01 %, wogegen das erfindungsgemäße Beispiel 1 eine Löslichkeit > 0,5 % aufweist.

Die besten PARP-Inhibitoren der Erfindung sind die der Beispiele 15, 16, 25, 36 und 37.

### Beispiele

### Beispiel 1

### 2-Pyridin-4-yl-benzimidazol-4-carbonsäureamid

a) 2-Pyridin-4-yl-benzimidazol-4-carbonsäureethylester
   1 g (5,5 mMol) 2,3-Diaminobenzoesäureethylester und 0,7 ml (11,3 mMol) Essigsäure wurden in 15 ml Ethanol gelöst. Danach wurden 0,77 g (7,2 mMol) Pyridin-4-aldehyd, gelöst in 25 ml Ethanol, innerhalb von 30 Minuten zugetropft. Anschließend wurde eine Lösung aus 1,44 g (7,2 mMol) Kupfer-II-sulfat in 20 ml Wasser zügig zugetropft. Alles wurde für 2 Stunden unter Rückfluß gekocht. Danach ließ man die Reaktionslösung auf 50°C abkühlen. Dann wurden 2,25 ml 32%iger Salzsäure zugegeben. Danach wurde eine Lösung aus 2,13 g (8,9 mMol) Natriumsulfid-Hydrat und 25ml Wasser vorsichtig in der Wärme zugetropft. Alles wurde noch für 10 Minuten gerührt. Der Reaktionsansatz wurde danach auf Eiswasser gegossen und der entstandene Niederschlag abfiltriert. Das Filtrat wurde mit wäßriger Natriumhydrogenkarbonat-Lösung alkalisch gestellt und mit Essigester extrahiert. Die organische Phase wurde mit Wasser gewaschen, getrocknet und im Vakuum eingeengt. Man erhielt 1,15 g des Produktes.
b) 2-Pyridin-4-yl-benzimidazol-4-carbonsäurehydrazid
   1 g (3,7 mMol) des Produktes 1a wurden in 30 ml Butanol gelöst. 6 ml Hydrazinhydrat wurden zugegeben und alles für 8 h unter Rückfluß gekocht. Nach dem Abkühlen wurde die Reaktionslösung im Vakuum eingeengt. Der Rückstand wurde mit Ether ausgerührt und abgesaugt, wobei man 0,74 g des Produktes erhielt.
c) 2-Pyridin-4-yl-benzimidazol-4-carbonsäureamid
   0,7 g (2,8 mMol) des Produktes 1b und 1,5 g Raney-Nickel (Aufschlämmung in Wasser) wurden in 45 ml Dimethylformamid/Wasser(2/1) für 8 Stunden auf 100°C erwärmt. Nach dem Abkühlen wurde filtriert und das Filtrat mit Wasser verdünnt, wobei ein Niederschlag ausfiel, der danach abgesaugt wurde. Man erhielt 0,16 g des Produktes.
   ¹H-NMR (D₆-DMSO): d = 7,4 (1H), 7,85 (2H), 7,9 (1H), 8,2 (2H), 8.8 (2H) und 9.2 (1H)ppm.

### Beispiel 2

### 2-Pyridin-4-yl-benzimidazol-4-carbonsäureamid x 2 Methansulfonsäure

61 mg (0,26 mMol) der Verbindung aus Beispiel 1 wurden in 1 ml Tetrahydrofuran gelöst und mit 25 mg (0,26 mMol) Methansulfonsäure, gelöst in 5 ml Wasser, versetzt. Anschließend wurde alles mit Wasser verdünnt und gefriergetrocknet. Man erhielt 58 mg des Produktes.
¹H-NMR (D₆-DMSO): δ = 2,6 (3H), 6,9 (1H), 7,1 (1H), 7,3-7,5 (3H), 7,8 (1H), 8,1 (1H), 8,8 (1H), 9,0 (1H) und 9,1 (1H) ppm.

### Beispiel 3

### 2-(Benzimidazol-5-yl)-benzimidazol-4-carbonsäureamid

a) 2-(Benzimidazol-5-yl)-benzimidazol-4-carbonsäure
   2 g (12 mMol) 2,3-Diaminobenzoesäuremethylester und 2 g (12 mMol) Benzimidazol-5-carbonsäure wurden nacheinander in 70 ml auf 90°C vorgewärmte Polyphosphorsäure eingetragen. Danach wurde alles für 1 Stunde auf 200°C erwärmt. Man kühlte anschließend den Reaktionsansatz auf 50 bis 60°C ab und goß ihn vorsichtig in Eiswasser. Der anfallende Niederschlag wurde abgesaugt und getrocknet. Man erhielt 2,7 g des Produktes.
b) 2-(Benzimidazol-5-yl)-benzimidazol-4-carbonsäureethylester
   2,6 g (9,3 mMol) des Produktes 3a wurden in 100 ml Ethanol gerührt und anschließend vorsichtig mit 10 ml konzentrierter Schwefelsäure versetzt. Alles wurde für 1 Stunde unter Rückfluß gekocht. Danach wurde die Reaktionslösung vorsichtig auf Eiswasser gegossen. Diese entstandene Lösung wurde mit wäßriger Natriumhydrogenkarbonat-Lösung alkalisch gestellt und mit Essigester extrahiert. Die organische Phase wurde abgetrennt, getrocknet und im Vakuum eingeengt. Man erhielt 2,7 g des Produktes.
c) 2-(Benzimidazol-5-yl)-benzimidazol-9-carbonsäurehydrazid
   2,6 g (8,5 mMol) des Produktes 3b wurden anlog dem Verfahren aus 1b mit Hydrazinhydrat umgesetzt. Man erhielt 1,4 g des Produktes.
d) 2-(Benzimidazol-5-yl)-benzimidazol-4-carbonsäureamid
   1,4 g des Produktes 3c wurden analog dem Verfahren aus 1c mit Raney-Nickel behandelt. Man erhielt 0,65 g des Produktes.
   ¹H-NMR (D₆-DMSO): δ = 7,3 (1H), 7,7-7,9 (5H), 8,2 (1H), 8,4 (1H), 8,5 (1H) und 9,5 (1H) ppm.

### Beispiel 4

### 2-(1-(2(N,N-Diethylamino)-1-ethyl)benzimidazol-5-yl)-benzimidazol-4-carbonsäureamid x 3 HCl

a) 1-(2(N,N-Diethylamino)-eth-1-yl)benzimidazol-5-carbonsäureethylester
   5,4 g (28,4 mMol) Benzimidazol-5-carbonsäureethylester, 9,8 g (56,8 mMol) N(2-Chlor-1-ethyl)-N,N-diethylamin und 7,9 g (56,8 mMol) Kaliumkarbonat wurden in 100 ml Dimethylformamid für 4 Stunden bei 100°C erwärmt. Anschließend wurde filtriert, das Filtrat im Vakuum eingeengt und der anfallende Rückstand chromatographisch (Fließmittel: Essigester/Aceton = 1/1) gereinigt. Man erhielt 2,6 g eines Isomerengemisches, in dem neben dem Produkt auch 3-(2(N,N-Diethylamino)-1-ethyl)-benzimidazol-5-carbonsäureethylester enthalten ist.
b) 1-(2 (N,N-Diethylamino)-eth-1-yl)benzimidazol-5-carbonsäure x 2 HCl
   2,5 g (8,6 mMol) des Produktes 4a wurden in 50 ml Ethanol gelöst, mit 50ml 1M Natronlauge versetzt und alles für 1 Stunde unter Rückfluß gekocht. Nach dem Abkühlen wurde die Reaktionslösung mit verdünnter Salzsäure neutralisiert und alles im Vakuum eingeengt. Der so erhaltene Rückstand wurde mit einem Gemisch aus Tetrahydrofuran und Methanol (1/1) ausgerührt und filtriert. Das Filtrat wurde im Vakuum eingeengt, anschließend in Wasser gelöst, mit zwei Äquivalenten Salzsäure versetzt und gefriergetrocknet. Man erhielt 3,4 g des Isomerengemisches.
c) 2-Amino-3(1-(2(N,N-diethylamino)-eth-2-yl)benzimidazol-5-yl)-amido-benzoesäure-methylester
   Zu 3,3 g (9,9 mMol) des Produktes 4b in 100 ml wasserfreiem Dimethylformamid wurden bei Raumtemperatur nacheinander 1,6 g (9,9 mMol) 2,3-Diaminobenzoesäuremethylester, 0,44 g (3,3 mMol) N-Hydroxybenzotriazol (HOBT) und 6,2 ml (44,4 mMol) Triethylamin gegeben. Anschließend wurden bei 15°C portionsweise 1,9 g (9,9 mMol) N'-(3-Dimethylaminopropyl)-N-ethylcarbodiimid (EDC) zugefügt. Alles wurde noch für 16 Stunden bei Raumtemperatur gerührt. Anschließend der Reaktionsansatz im Vakuum eingeengt. Der Rückstand wurde zwischen wasser und Essigester verteilt. Die wäßrige Phase wurde abgetrennt, mit wäßriger Natriumhydrogenkarbonat-Lösung alkalisiert und mit Essigester extrahiert. Diese organische Phase wurde abgetrennt, mit Aktivkohle behandelt, filtriert, getrocknet und im Vakuum eingeengt. Man erhielt 1,5 g des Produktes als Isomerengemisch.
d) 2-(1-(2(N,N-Diethylamino)-eth-1-yl)benzimidazol-5-yl)-benzimidazol-4-carbonsäure-methylester
   1,5 g des Produktes 4c wurden in 75 ml Essigsäure für 1 Stunde unter Rückfluß gekocht. Anschließend wurde alles im Vakuum eingeengt. Man erhielt 2,2 g des Produktes.
e) 2-(1-(2(N,N-Diethylamino)-eth-1-yl)benzimidazol-5-yl)-benzimidazol-4-carbonsäurehydrazid
   2,2 g des Produktes 4d wurden analog dem Verfahren 1b mit Hydrazinhydrat umgesetzt. Man erhielt ein Rohprodukt, das ungereinigt weiter umgesetzt wurde.
f) 2-(1-(2(N,N-Diethylamino)-eth-1-yl)benzimidazol-5-yl)-benzimidazol-4-carbonsäureamid x 3 HCl
   Das Produkt aus 4e wurde analog dem Verfahren 1c mit Raney-Nickel behandelt. Das Rohprodukt wurde zuletzt in warmen Isopropanol gelöst und mit wenig isopropanolische Chlorwasserstoff-Lösung versetzt. Beim Abkühlen kristallisierte das Produkt aus. Man erhielt 0,98 g des Isomerengemisches.
   MS: m/e = 376 (M⁺) .

### Beispiel 5

### 2-(1(2(N,N-Diethylamino)-eth-1-yl)indol-3-yl)-benzimidazol-4-carbonsäureamid

a) 1-(2-N,N-Diethylamino-eth-1-yl)indol-3-aldehyd
   Zu einer Lösung aus 5 g (34,5 mMol) Indol-3-aldehyd in 100 ml wasserfreiem Tetrahydrofuran gab man bei 0°C portionsweise 1,1 g (45,4 mMol) Natriumhydrid (80%ig) zu. Alles wurde noch für 15 Minuten gerührt. Anschließend wurden 7,4 g (68,9 mMol) N(2-Chlor-1-ethyl)-N,N-diemthylamin, gelöst in 50 ml wasserfreiem Tetrahydrofuran, tropfenweise zugegeben. Anschließend wurde alles noch für 16 Stunden bei Raumtemperatur gerührt. Danach wurde die Reaktionslösung tropfenweise (Achtung Natriumhydrid!) mit 40 ml Wasser versetzt und das organische Lösungsmittel im Vakuum entfernt. Die zurückbleibende Phase wurde mit Wasser verdünnt und mit Ether extrahiert. Diese organische Phase wurde mit 2 M Salzsäure und Wasser gewaschen, getrocknet und im Vakuum eingeengt.
b) 2-(1(2(N,N-Diethylamino)-eth-1-yl)indol-3-yl)-benzimidazol-4-carbonsäuremethylester
   1,9 g (7,8 mMol) des Produktes 5a und lg (6 mMol) 2,3-Diaminobenzoesäuremethylester wurden analog der Vorschrift 1a umgesetzt. Man erhielt 1,5 g des Produktes.
c) 2-(1(2(N,N-Diethylamino)-eth-1-yl)indol-3-yl)-benzimidazol-4-carbonsäurehydrazid
   1,5 g des Produktes 5b wurden anlog der Vorschrift 1b mit Hydrazinhydrat umgesetzt. Man erhielt 0,39 g des Produktes.
d) 2-(1(2(N,N-Diethylamino)-eth-1-yl)indol-3-yl)-benzimidazol-4-carbonsäureamid
   0,35 g des Produktes 5c wurden anlog der Vorschrift 1c mit Raney-Nickel behandelt. Man erhielt 0,14 g des Produktes. ¹H-NMR (D₆-DMSO) : δ = 1,1 (3H), 2,8-3,2 (6H), 4,6 (2H), 7,2.-7,4 (3H), 7,6-7,9 (4H), 8,4 (2H) und 9,6 (1H)ppm.

### Beispiel 6:

### 2-(Pyrazin-2-yl)-benzimidazol-4-carbonsäureamid

Eine Lösung von 1.68 g (7.5 mmol) 2,3-Diaminobenzoesäureamid und 3.04 g (30 mmol) Triethylamin in 50 ml Dimethylformamid wurde 1 Stunde bei Raumtemp. gerührt. 0.93 g (7.5 mmol) Pyrazin-2-carbonsäure und 1.01 g (7.5 mmol) N-Hydroxybenzotriazol (HOBt) wurden zugegeben. Nach 15 Minuten Rühren bei Raumtemp. wurde die Mischung auf 0°C gekühlt. 1.44 g (7.5 mmol) N'-(3-Dimethylamino-propyl)-N-ethylcarbodiimid (EDC) wurden zugegeben, und die Mischung wurde 16 Stunden bei Raumtemp. gerührt. Anschließend wurde der Reaktionsansatz im Vakuum eingeengt. Der Rückstand wurde zwischen Methylenchlorid und gesättigter Natriumhydrogencarbonat-Lösung verteilt. Die wäßrige Phase wurde mit Methylenchlorid extrahiert. Die vereinigten organischen Phasen wurden über Magnesiumsulfat getrocknet. Nach Einengen im Vakuum wurde der erhaltene Rückstand in 50 ml Eisessig aufgenommen und 30 Minuten unter Rückfluß gekocht. Der Reaktionsansatz wurde im Vakuum eingeengt und der Rückstand in Diethylether gerührt. Der Niederschlag wurde durch Filtration abgetrennt und bei 40°C im Vakuum getrocknet. 1.11 g braune Kristalle wurden erhalten.
1H-NMR (D₆-DMSO): δ = 7.42 (1H), 7.6-7.9 (2H), 7.95 (1H), 8.8-8.9 (2H), 9.2 (1H), 9.7 (1H) ppm.
MS: m/e = 239 (M⁺)

Analog Beispiel 6 wurden die Beispiele 7-11 hergestellt:

### Beispiel 7:

2-(Chinolin-6-yl)-benzimidazol-4-carbonsäureamid
1H-NMR (D₆-DMSO): δ = 7.4 (1H), 7.55-8.2 (4H), 8.2-8.3 (1H), 8.4-8.7 (2H), 8.8-9.2 (2H), 9.3 (breit, 1H), 13.0 (breit, 1H) ppm.
MS: m/e = 288 (M⁺)

### Beispiel 8:

### 2-(1-(2-(N,N-Diethylamino)-eth-1-yl)-pyrrol-3-yl)-benzimidazol-4-carbonsäureamid

1H-NMR (D₆-DMSO): **δ** = 0.8-1.0 (6H), 2.4-2.6 (4H), 2.7-2.8 (2H), 4.0-4.1 (2H), 6.7-6.75 (1H), 7.1-7.15 (1H), 7.5-7.8 (3H), 8.0-8.2 (2H) ppm.
MS: m/e = 326 (M⁺+H)

### Beispiel 10:

### 2-Pyridin-3-yl-benzimidazol-4-carbonsäureamid

1H-NMR (D₆-DMSO): δ = 7.25 (1H), 7.5-7.7 (3H), 7.75 (1H), 7.8-8.0 (2H), 7.25-7.3 (1H), 7.4 (1H) ppm.
MS: m/e = 238 (M⁺)

### Beispiel 11:

### 2-(2-Aminomethyl-thiazol-4-yl)-benzimidazol-4-carbonsäureamid × HCl

1H-NMR (D₆-DMSO): δ = 4.0-5.0 (breit, NH), 4.75 (1H), 4.8 (1H), 7.5 (1H), 7.8 (1H), 7.95 (1H), 9.0 (breit) ppm.
MS: m/e = 273 (M⁺)

### Beispiel 12:

### 2-Isoxazol-5-yl-benzimidazol-4-carbonsäureamid

Zu einer Lösung von 0.85 g (7.5 mmol) Isoxazol-5-carbonsäure und 3.79 g (37.5 mmol) Triethylamin in 50 ml Tetrahydrofuran wurde bei -10°C eine Lösung von 0.81 g (7.5 mmol) Chlorameisensäureethylester in 5 ml Tetrahydrofuran zugetropft. Nach 1 Stunde Rühren bei -10°C wurden 1.68 g (7.5 mmol) 2,3-Diaminobenzoesäureamid zugegeben. Die Reaktionsmischung wurde 16 Stunden bei Raumtemp. gerührt. Anschließend wurde der Reaktionsansatz im Vakuum eingeengt. Der Rückstand wurde zwischen Methylenchlorid und gesättigter Natriumhydrogencarbonat-Lösung verteilt. Die wäßrige Phase wurde mit Methylenchlorid extrahiert. Die vereinigten organischen Phasen wurden über Magnesiumsulfat getrocknet. Nach Einengen im Vakuum wurde der erhaltene Rückstand in 10 ml Eisessig aufgenommen und 60 Minuten bei 100°C gerührt. Der Reaktionsansatz wurde im Vakuum eingeengt und der Rückstand zwischen Ethylacetat und gesättigter Natriumhydrogencarbonat-Lösung verteilt. Die organische Phase wurde über Magnesiumsulfat getrocknet und im Vakuum eingeengt. Der Rückstand wurde in Isopropanol aufgenommen, und der mit Petrolether gefällte Niederschlag wurde filtriert, mit Petrolether gewaschen und im Vakuum bei 35°C getrocknet. 120 mg gelbe Kristalle wurden erhalten.
1H-NMR (D₆-DMSO): δ = 7.3 (1H). 7.45-7.5 (1H), 7.75-8.0 (3H), 8.8 (1H), 9.1 (1H) ppm.
MS: m/e = 228 (M⁺)

### Beispiel 13:

### 2-(2-(2-(N,N-Diethylamino)-eth-1-yl-amino)-pyridin-3-yl)-benzimidazol-4-carbonsäureamid × 2HCl

a) 2-(2-(N,N-Diethylamino)-eth-1-yl-amino)-nicotinsäureethylester × 2 Oxalsäure
   Eine Mischung von 1.86 g (0.01 mol) 2-Chlornicotinsäureethylester, 1.16 g (0.01 mol) 1-(N,N-Diethylamino)-2-aminoethan, 2.76 g (0.02 mol) Kaliumcarbonat und eine Spatelspitze 18-Krone-6 in 50 ml Dimethylformamid wurde 6 Stunden bei 120°C gerührt. Nach Abtrennen der Feststoffe wurde der Reaktionsansatz im Vakuum eingeengt. Der Rückstand wurde in Ethylacetat aufgenommen. Die organische Phase wurde mit Wasser gewaschen, über Magnesiumsulfat getrocknet und im Vakuum eingeengt. Das hellbraune Öl wurde in Isopropanol aufgenommen, und mit Oxalsäure wurde das Oxalat gefällt. Nach Abtrennen und Trocknen im Vakuum wurden 2.2 g weiße Kristalle erhalten.
b) 2-(2-(N,N-Diethylamino)-eth-1-yl-amino)-nicotinsäure
   1.15 g (4.33 mmol) der freien Base des Produktes 13a wurden in 100 ml Methanol vorgelegt und mit 100 ml 2M Natronlauge versetzt. Die Reaktionsmischung wurde 16 Stunden bei Raumtemp. gerührt. Der Reaktionsansatz wurde im Vakuum eingeengt, und der wäßrige Rückstand wurde mit 100 ml 2M Salzsäure versetzt. Nach Einengen im Vakuum wurden 12.47 g eines Gemisches aus Produkt und NaCl erhalten.
c) 2-(2-(2-(N,N-Diethylamino)-eth-1-yl-amino)-pyridin-3-yl)-benzimidazol-4-carbonsäureamid × 2HCl
   0.84 g (3.75 mmol) 2,3-Diaminobenzoesäureamid wurden in 35 ml Pyridin 5 Minuten gerührt, dann wurden 0.89 g (3.75 mmol) Produkt 13b zugegeben. Die Reaktionsmischung wurde 5 Minuten gerührt und auf 0°C gekühlt. Nach Zugabe von 0.72 g (3.75 mmol) N'-(3-Dimethylamino-propyl)-N-ethylcarbodiimid (EDC) wurde die Reaktionsmischung 1 Stunde bei 0°C und anschließend 16 Stunden bei Raumtemp. gerührt. Der Reaktionsansatz wurde im Vakuum eingeengt und anschließend zweimal mit Toluol im Vakuum eingeengt. Der Rückstand wurde zwischen Wasser und Ethylacetat verteilt. Die wäßrige Phase wurde mit 1M Natronlauge basisch gestellt, mit festem Natriumchlorid gesättigt und mit Methylenchlorid erschöpfend extrahiert. Die organische Phase wurde mit Magnesiumsulfat getrocknet und im Vakuum eingeengt. Der Rückstand wurde in 2 ml Eisessig aufgenommen und 1 Stunde bei 100°C gerührt. Nach Einengen im Vakuum wurde der Rückstand in Methylenchlorid gelöst, und mit etherischer Hydrochloridlösung wurde das Hydrochlorid gefällt. Dieses wurde durch Filtration abgetrennt, mit Diethylether gewaschen und im Vakuum bei 35°C getrocknet. Es wurden 0.52 g eines gelben Pulvers erhalten.
1H-NMR (D₆-DMSO): δ = 1.1-1.3 (6H), 3.1-3.3 (4H), 3.35-3.5 (2H), 4.0-4.15 (2H), 7.0-7.1 (1H), 7.4-7.5 (1H), 7.8-7.95 (2H), 8.2-8.3 (1H), 8.4 (1H), 8.7 (1H), 10.6 (1H) ppm.
MS: m/e = 353 (M⁺+H)

### Beispiel 14:

### 2-(2-((2-(N,N-Diethylamino)-eth-1-yl)-methylamino)-pyridin-3-yl)-benzimidazol-4-carbonsäureamid × 2HCl

Die Synthese erfolgte analog zu Beispiel 13.
1H-NMR (D₆-DMSO): δ =1.25 (6H), 2.6 (3H), 3.1-3.25 (4H), 3.3-3.45 (2H), 3.8- 3.9 (2H), 7.0-7.1 (1H), 7.45-7.55 (1H), 7.8 (1H), 7.95-8.05 (2H), 8.15-8.2 (1H), 8.4-8.45 (1H), 8.8 (breit, 2H), 10.55 (breit, 1H) ppm.
MS: m/e = 367 (M⁺+H)

### Beispiel 15:

### 2-(6-((2-(N,N-Diethylamino)-eth-1-yl)-methylamino)-pyridin-3-yl)-benzimidazol-4-carbonsäureamid × 2 HCl

Die Synthese erfolgte analog zu Beispiel 13.
1H-NMR (D₄-Methanol): δ = 1.45 (6H), 3.25 (3H), 3.3-3.5 (6H), 4.2 (2H), 7.1 (1H), 7.7 (1H), 7.95 (1H). 8.05 (1H), 8.35 (1H), 8.7 (1H) ppm.
MS: m/e = 367 (M⁺+H)

### Beispiel 16:

### 2-(6-(4-Propyl-piperazin-1-yl)-pyridin-3-yl)-benzimidazol-4-carbonsäureamid

Die Synthese erfolgte analog zu Beispiel 13.
1H-NMR (D₆-DMSO): δ = 0.95 (3H), 1.7-1.8 (2H), 2.7-2.8 (2H), 3.0-3.2 (6H), 4.5-4.65 (2H), 7.15-7.25 (1H), 7.4 (1H), 7.75-7.95 (2H), 8.45 (1H), 9.05 (1H), 10.95 (breit, 1H) ppm.
MS: m/e = 365 (M⁺+H)

### Beispiel 17:

### 2-(2-(3-(N,N-Diethylamino)-prop-1-yl-amino)-pyridin-3-yl)-benzimidazol-4-carbonsäureamid × 2 HCl

Die Synthese erfolgte analog zu Beispiel 13.
1H-NMR (D₄-Methanol): δ = 1.2 (6H), 2.0-2.1 (2H), 2.85-3.0 (6H), 3.6-3.7 (2H), 7.7-7.8 (1H), 7.3-7.4 (1H), 8.1-8.2 (2H), 7.75 (1H), 7.9 (1H) ppm.
MS: m/e = 367 (M⁺+H)

### Beispiel 18:

### 2-(3-Amino-thiophen-4-yl)-benzimidazol-4-carbonsäureamid × HCl

a) 4-*tert*.-Butyloxycarbonylamino-thiophen-3-carbonsäure
   2.76 g (0.022 mol) 4-Amino-thiophen-3-carbonsäureethylester und 4.81 g (0.022 mol) Di-*tert*.-butyldicarbonat wurden mit einer Spatelspitze 4-N,N-Dimethylaminopyridin in 100 ml Tetrahydrofuran 8 Stunden bei Raumtemp. gerührt. Der Reaktionsansatz wurde mit Ethylacetat verdünnt, dreimal mit 5% Zitronensäure-Lösung gewaschen. Nach Trocknen mit Magnesiumsulfat wurde die organische Phase im Vakuum eingeengt. Der Rückstand wurde in 100 ml Ethanol gelöst, 100 ml 2M Natronlauge wurden zugegeben, und die Mischung wurde 16 Stunden bei Raumtemp. gerührt. Nach Einengen im Vakuum wurde der wäßrige Rückstand mit 100 ml 2M Salzsäure versetzt. Der Niederschlag wurde durch Filtration abgetrennt und mit Wasser gewaschen und bei 40°C im Vakuum getrocknet. Der Rückstand wurde mit Ethylacetat ausgerührt. Nach Filtration wurde das Filtrat im Vakuum eingeengt. 0.85 g gelbes Pulver wurden erhalten.
b) 2-(3-Amino-thiophen-4-yl)-benzimidazol-4-carbonsäureamid × HCl
   Zu einer Lösung von 0.78 g (3.21 mmol) Produkt 18a und 1.67 g (16.05 mmol) Triethylamin in 25 ml Methylenchlorid wurde bei -10°C eine Lösung von 0.35 g (3.21 mmol) Chlarameisensäureethylester in 5 ml Methylenchlorid zugetropft. Nach 1 Stunde Rühren bei -10°C wurden 0.72 g (3.21 mmol) 2,3-Diaminobenzoesäureamid zugegeben. Die Reaktionsmischung wurde 16 Stunden bei Raumtemp. gerührt. Anschließend wurde der Reaktionsansatz im Vakuum eingeengt. Der Rückstand wurde zwischen Methylenchlorid und gesättigter Natriumhydrogencarbonat-Lösung verteilt. Die wäßrige Phase wurde mit Methylenchlorid extrahiert. Die vereinigten organischen Phasen wurden über Magnesiumsulfat getrocknet. Nach Einengen im Vakuum wurde der erhaltene Rückstand in 10 ml Eisessig aufgenommen und 30 Minuten bei 100°C gerührt. Der Reaktionsansatz wurde im Vakuum eingeengt und der Rückstand in wenig Ethylacetat aufgenommen. Der mit n-Pentan gefällte Niederschlag wurde filtriert und in 5 ml Methylenchlorid aufgenommen. Nach Zugabe von 5 ml 4M Dioxan-HCl-Lösung wurde die Mischung 16 Stunden bei Raumtemp. gerührt. Das Kristallisat wurde filtriert, mit Methylenchlorid und Diethylether gewaschen und bei 35 °C im Vakuum getrocknet. Man erhielt 30 mg ockerfarbene Kristalle.
   1H-NMR (D₆-DMSO): **δ** = 7.3-7.4 (1H), 7.7-7.8 (2H), 7.85-7.9 (1H), 8.6 (1H), 8.75 (1H) ppm.
   MS: m/e = 258 (M⁺)

### Beispiel 19:

### 2-(2-(2-(N,N-Diethylamino)-eth-1-yl-oxy)-pyridin-3-yl)-benzimidazol-4-carbonsäureamid × HCl

a) 2-(2-(N,N-Diethylamino)-eth-1-yl-oxy)-nicotinsäureethylester × 2 Oxalsäure
   1.05 g (6.28 mmol) 2-Hydroxynicotinsäureethylester, 1.64 g (6.28 mmol) 2-Brom-N,N-diethyl-ethylenamin × HBr, 1.74 g Kaliumcarbonat und eine Spatelspitze 18-Krone-6 in 25 ml Dimethylformamid wurden 16 Stunden bei Raumtemp. gerührt. Anschließend wurden die Feststoffe abgetrennt, das Filtrat wurde im Vakuum eingeengt. Der Rückstand wurde zwischen Wasser und Ethylacetat verteilt. Die organische Phase wurde über Magnesiumsulfat getrocknet und im Vakuum eingeengt. Der Rückstand wurde in Isopropanol aufgenommen, und mit Oxalsäure wurde das Oxalat gefällt. Dieses wurde nach Abtrennen bei 40°C im Vakuum getrocknet. Es wurden 2.2 g weiße Kristalle erhalten.
b) 2-(2-(N,N-Diethylamino)-eth-1-yl-oxy)-nicotinsäure
   Analog Beispiel 13b wurden 0.87 g (3.27 mmol) Produkt 21a umgesetzt. Es wurden 3.54 g eines Gemisches aus Produkt 18b und NaCl erhalten.
c) 2-(2-(2-(N,N-Diethylamino)-eth-1-yl-oxy)-pyridin-3-yl)-benzimidazol-4-carbonsäureamid × HCl
   Analog Beispiel 13c wurden 1.68 g (7.5 mmol) 2,3-Diaminobenzoesäureamid und 1.79 g (7.5 mmol) Produkt 21b umgesetzt. Es wurden 390 mg eines ockerfarbenen Pulvers erhalten.
   1H-NMR (D₆-DMSO): δ = 1.2-1.3 (6H), 3.2-3.3 (4H), 3.4-3.5 (2H), 4.5-4.6 (2H), 6.7-6.8 (1H), 7.4-7.5 (1H), 7.8 (1H), 7.9-8.1 (2H), 8.3-8.4 (1H), 8.7 (breit, 1H), 8.9-9.0 (1H), 10.8 (breit, 1H) ppm.
   MS: m/e = 353 (M⁺)

### Beispiel 20:

### 2-(1-Phenylsulfonyl)-pyrrol-3-yl-benzimidazol-4-carbonsäureamid

a) 1-Phenylsulfonyl-pyrrol-3-carbaldehyd
   Eine Lösung von 5.91 g (0.037 mol) 2,5-Dimethoxy-tetrahydrofuran-3-carbaldehyd, 5.80 g (0.037 mol) Phenylsulfonamid und eine Spatelspitze 4-Toluolsulfonsäure in 50 ml Toluol wurden am Wasserabscheider unter Rückfluß gekocht bis kein Wasser mehr abgeschieden wurde. Der Reaktionsansatz wurde dreimal mit Wasser gewaschen. Die organische Phase wurde über Magnesiumsulfat unter Zusatz von Aktivkohle und Kieselgel getrocknet und im Vakuum eingeengt. Man erhielt 7.68 g braunes Harz.
b) 2-(1-Phenylsulfonyl)-pyrrol-3-yl-benzimidazol-4-carbonsäureamid
   Zu einer Lösung von 0.84 g (15 mmol) Kaliumhydroxid-Pulver in 100 ml Ethanol wurden 1.68 g (7.5 mmol) 2,3-Diaminobenzoesäureamid gegeben. Nach 5 Minuten Rühren wurden schnell 1.35 g (22.5 mmol) Eisessig und innerhalb von 30 Minuten eine Lösung von Produkt 20a in 20 ml Ethanol zugetropft. Anschließend wurde eine warme Lösung von 2.49 g (12.5 mmol) Kupfer-II-acetat in 20 ml Wasser rasch zugetropft. Der Reaktionsansatz wurde 2 Stunden unter Rückfluß gekocht. Anschließend wurden feste Bestandteile durch Filtration abgetrennt. Der Rückstand wurde zwischen Methylenchlorid und Wasser verteilt. Mit Ammoniakwasser wurde die Mischung alkalisch gestellt. Die wäßrige Phase wurde mit Methylenchlorid extrahiert. Die vereinigten organischen Phasen wurden über Magnesiumsulfat getrocknet und im Vakuum eingeengt. Man erhielt 0.82 g gelbe Kristalle. 1H-NMR (D₆-DMSO): δ = 7.05 (1H), 7.3-7.35 (1H), 7.55-7.65 (2H), 7.65-7.75 (5H), 7.8-7.9 (2H), 8.0-8.1 (2H), 8.2 (1H), 9.3 (1H) ppm.
   MS: m/e = 366 (M⁺)

### Beispiel 21:

### 2-Pyrrol-3-yl-benzimidazol-4-carbonsäureamid

Eine Mischung aus 0.5 g (1.36 mmol) Produkt 20b und 0.75 g Kaliumcarbonat in 25 ml Methanol und 10 ml Wasser wurde 2 Stunden unter Rückfluß erhitzt. Anschließend wurde der Reaktionsansatz im Vakuum eingeengt. Der Rückstand wurde mit gesättigter Natriumhydrogencarbonat-Lösung verdünnt und mehrfach mit Ethylacetat extrahiert. Nach Trocknen über Magnesiumsulfat wurde im Vakuum eingeengt. Der Rückstand wurde in wenig Ethylacetat/Tetrahydrofuran gelöst, mit n-Pentan wurde das Produkt gefällt. Dieses wurde durch Filtration gewonnen und bei 30°C im Vakuum getrocknet. Es wurden 80 mg gelbe Kristalle erhalten.
1H-NMR (D₆-DMSO): δ = 6.75 (1H), 6.9 (1H), 7.2-7.3 (1H), 7.5-7.7 (2H), 7.7-7.8 (2H), 9.4 (breit, 1H), 11.3 (breit, 1H) ppm.
MS: m/e = 226 (M⁺+H)

### Beispiel 22:

### 2-(2-Methyl-imidazo[1,5-a]pyridin-8-yl)-benzimidazol-4-carbonsäureamid

Die Synthese erfolgte analog Beispiel 20b.
1H-NMR (D₆-DMSO): δ = 2.7 (3H), 5.8 (breit, NH), 7.0-7.4 (5H), 8.4 (1H), 8.55 (1H), 10.9 (1H) ppm.
MS: m/e = 291 (M⁺)

### Beispiel 23:

### 2-(Pyrazol-4-yl)-benzimidazol-4-carbonsäureamid

0.56 g (5 mmol) 4-Pyrazolcarbonsäure wurden mit 0.81 g (5 mmol) Carbonyldiimidazol in 20 ml Dimethylformamid 2 Stunden bei Raumtemp. gerührt. 1.12 g (5 mmol) 2,3-Diaminobenzoesäureamid in 10 ml Pyridin wurden zugegeben. Der Reaktionsansatz wurde 2 Stunden bei 50°C gerührt. Anschließend wurde der Reaktionsansatz im Vakuum eingeengt und der Rückstand mit Wasser verrührt. Der ausgefallene Niederschlag wurde abfiltriert bei 40°C im Vakuum getrocknet und anschließend in 10 ml Eisessig bei 100°C gerührt. Nach Einengen im Vakuum wurde der Rückstand mit gesättigter Natriumhydrogencarbonat-Lösung verrührt. Nach Filtration wurde das Filtrat mehrmals mit Toluol im Vakuum eingeengt. Der Rückstand wurde mit Tetrahydrofuran ausgerührt. Nach Einengen im Vakuum wurden 150 mg gelbes Pulver erhalten.
1H-NMR (D₆-DMSO): δ = 7.2 (1H), 7.55-7.7 (3H). 7.75 (1H), 8.4 (2H), 9.3 (breit, 1H) ppm.
MS: m/e = 291 (M⁺)

### Beispiel 24:

### 2-(2-(3-(N,N-Diethylamino)-prop-1-yl-amino)-pyridin-4-yl)-benzimidazol-4-carbonsäureamid

a) 2-(3-(N,N-Diethylamino)-prop-1-yl-amino)-isonicotinsäuretert.butylester
   1.02 g (4.77 mmol) 2-Chlorisonicotinsäure-tert.-butylester, 5 ml 3-Diethylamino-1-propylamin, 0.66 g (4.77 mmol) Kaliumcarbonat, eine Spatelspitze Cu-Pulver und eine Spatelspitze 18-Krone-6 wurden 4 Stunden unter Rückfluß gekocht. Durch Flashchromatographie der Reaktionsmischung (Toluol/Tetrahydrofuran/Methanol, 4/1/1 + 2.5% Triethylamin) wurden 0.69 g hellbraunes Öl erhalten.
b) 2-(3-(N,N-Diethylamino)-prop-1-yl-amino)-isonicotinsäure × HCl
   Eine Lösung von 0.5 g (1.63 mmol) Produkt 24a in 7.5 ml Dioxan und 7.5 ml 2M Salzsäure wurde 1 Stunde bei 100°C gerührt. Nach Einengen des Reaktionsansatzes im Vakuum und Trocknen des Rückstandes im Vakuum bei 50°C wurden 0.43 g beige Kristalle erhalten
c) 2 -(2-(3-(N,N-Diethylamino)-prop-1-yl-amino)-pyridin-4-yl)-benzimidazol-4-carbonsäureamid
   0.36 g (1.25 mmol) Produkt 24b wurden in 10 ml einer Mischung aus Dimethylformamid und Pyridin (1:1) vorgelegt und 15 Minuten gerührt. Nach Zugabe von 0.21 g (1.31 mmol) Carbonyldiimidazol wurde die Reaktionsmischung 1 Stunde bei Raumtemp. gerührt. Anschließend wurde eine Lösung von 0.28 g (1.25 mmol) 2,3-Diaminobenzoesäureamid in 5 ml Dimethylformamid/Pyridin zugegeben. Die Reaktionsmischung wurde jetzt 2 Stunden bei 50°C gerührt. Nach Einengen des Reaktionsansatzes im Vakuum wurde der Rückstand zwischen Ethylacetat und gesättigter Natriumhydrogencarbonat-Lösung verteilt. Die organische Phase wurde abgetrennt, über Magnesiumsulfat getrocknet und im Vakuum eingeengt. Der Rückstand wurde in 20 ml Eisessig 1 Stunde bei 100°C gerührt. Nach Einengen des Reaktionsansatzes im Vakuum wurde der Rückstand durch Flashchromatographie gereinigt (Toluol/Tetrahydrofuran/Methanol, 4/1/1 + 2.5% Triethylamin). Das so gewonnene Produkt wurde in wenig Aceton gelöst. Der mit Diethylether erhaltene Niederschlag wurde abgetrennt und im Vakuum bei 40°C getrocknet. Es wurden 0.22 g gelbes Pulver erhalten.
   1H-NMR (D₆-DMSO): δ = 1.15-1.25 (6H), 1.9-2.05 (2H), 3.0-3.2 (8H), 7.1 (1H), 7.3-7.35 (2H), 7.8 (1H), 7.9 (1H), 7.95 (1H), 8.2 (1H), 9.3 (1H), 14.0 (1H) ppm.
   MS: m/e = 366 (M⁺)

### Beispiel 25:

### 2-(2-((2-(N,N-Diethylamino)-eth-1-yl)-methylamino)-pyridin-4-yl)-benzimidazol-4-carbonsäureamid × ½ Fumarsäure

Die Synthese erfolgte analog Beispiel 24.
1H-NMR (D₆-DMSO): δ = 1.2-1.35 (6H), 3.2 (3H), 3.2-3.5 (4H), 3.95-4.05 (4H), 6.65 (2H), 7.4 (1H), 7.5 (2H), 7.6 (1H), 7.75-7.85 (2H), 7.9 (1H), 8.3 (1H), 9.25 (1H), 14.0 (1H) ppm.
MS: m/e = 366 (M⁺)

### Beispiel 26:

### 2-(2-(2-(N,N-Diethylamino)-eth-1-yl-amino)-pyridin-4-yl)-benzimidazol-4-carbonsäureamid × 2 HCl

Die Synthese erfolgte analog Beispiel 24.
1H-NMR (D₆-DMSO): δ = 1.0-1.05 (6H), 3.3-3.45 (4H), 3.9-4.1 (4H), 6.65 (1H), 7.25-7.4 (2H), 7.5 (1H), 7.55-7.65 (2H), 7.75 (1H), 7.85 (1H), 7.9-8.0 (1H), 8.05 (1H), 8.1-8.25 (2H), 9.0 (1H), 10.55 (breit, 1H) ppm.
MS: m/e = 353 (M⁺+H)

### Beispiel 27:

### 2-(2-(2-(Pyrrolidin-1-yl)-eth-1-yl-amino)-pyridin-4-yl)-benzimidazol-4-carbonsäureamid

Die Synthese erfolgte analog Beispiel 24.
1H-NMR (D₆-DMSO): δ = 1.7-1.8 (4H), 2.55-2.7 (4H), 2.75 (2H), 3.5 (2H), 6.9 (1H), 7.25 (1H), 7.3-7.45 (2H), 7.75 (1H), 7.85-7.95 (2H), 8.15 (1H), 9.25 (1H) ppm.
MS: m/e = 351 (M⁺)

### Beispiel 28:

### 2-(2-(3-(4-Methyl-piperazin-1-yl)-prop-1-yl-amino)-pyridin-4-yl)-benzimidazol-4-carbonsäureamid × 2 Fumarsäure

Die Synthese erfolgte analog Beispiel 24.
1H-NMR (D₆-DMSO): δ = 2.7-2.85 (2H), 2.45-2.8 (13H), 3.25-3.4 (2H). 6.6 (4H), 6.95 (breit, 1H), 7.25 (1H), 7.3 (1H), 7.4 (1H), 7.8 (1H), 7.85-7.95 (2H), 8.15 (1H), 9.25 (breit, 1H), 13.8 (breit, 1H) ppm.
MS: m/e = 394 (M⁺+H)

### Beispiel 29:

### 2-(6-(2-(Pyrrolidin-1-yl)-eth-1-yl-amino)-pyridin-2-yl)-benzimidazol-4-carbonsäureamid

Die Synthese erfolgte analog Beispiel 24.
1H-NMR (D₆-DMSO): δ = 1.65-1.8 (4H), 2.5-2.6 (4H), 2.65-2.75 (2H), 3.55-3.7 (2H), 6.65-6.75 (2H), 7.35 (1H), 7.5-7.6 (2H), 7.75-7.85 (2H), 7.9 (1H), 9.3 (breit, 1H) ppm.
MS: m/e = 351 (M⁺+H)

### Beispiel 30:

### 2-(6-(3-(N,N-Diethylamino)-prop-1-yl-amino)-pyridin-2-yl)-benzimidazol-4-carbonsäureamid

Die Synthese erfolgte analog Beispiel 24.
1H-NMR (D₆-DMSO): δ =
MS: m/e = 367 (M⁺+H)

### Beispiel 31:

### 2-(6-(2-(N,N-Diethylamino)-eth-1-yl-amino)-pyridin-2-yl)-benzimidazol-4-carbonsäureamid × ½ Fumarsäure

Die Synthese erfolgte analog Beispiel 24.
1H-NMR (D₆-DMSO): δ = 0.95-1.1 (6H), 2.65-2.8 (6H), 3.55-3.7 (2H), 6.55 (1H), 6.7 (1H) 7.3 (1H), 7.5-7.6 (2H), 7.7-7.8 (2H), 7.9 (1H), 9.3 (breit), 1H) ppm.
MS: m/e = 353 (M⁺+H)

### Beispiel 32:

### 2-(6-(3-(4-Methyl-piperazin-1-yl)-prop-1-yl-amino)-pyridin-2-yl)-benzimidazol-4-carbonsäureamid

Die Synthese erfolgte analog Beispiel 24.
MS: m/e = 394 (M⁺+H)

### Beispiel 33:

### 2-(6-(2-((N,N-Diethylamino)-eth-1-yl)-methylamino)-pyridin-2-yl)-benzimidazol-4-carbonsäureamid × 3 Fumarsäure

Die Synthese erfolgte analog Beispiel 24.
1H-NMR (D₆-DMSO): δ = 1.05-1.2 (6H), 3.0-3.1 (6H), 3.15 (3H), 4.0-4.15 (2H), 6.6 (6H), 6.75-6.85 (2H), 7.05 (breit, 1H), 7.35 (1H), 7.65-7.8 (4H), 7.9 (1H), 8.2 (breit, 1H), 9.3 (breit, 1H) ppm.
MS: m/e = 367 (M⁺+H)

### Beispiel 34:

### 2-(6-(4-Phenyl-piperazin-1-yl)-pyridin-3-yl)-benzimidazol-4-carbonsäureamid × 2 HCl

a) 6-(4-Phenyl-piperazin-1-yl)-nicotinsäureethylester
   Eine Mischung aus 3.72 g (0.02 mol) 6-Chlor-nicotinsäureethylester und 6.44 g (0.04 mol) N-Phenylpiperazin wurde 1 Stunde bei 100°C gerührt. Der Reaktionsansatz wurde im Vakuum eingeengt. Der Rückstand wurde mit Isopropanol ausgerührt. Es wurden 7.8 g eines gelben Feststoffes erhalten.
b) 6-(4-Phenyl-piperazin-1-yl)-nicotinsäure
   Analog Beispiel 13b wurden 2.8 g Produkt 34b erhalten.
c) 2-(6-(4-Phenyl-piperazin-1-yl)-pyridin-3-yl)-benzimidazol-4-carbonsäureamid
   Analog Beispiel 24c wurden 2.27 g (0.008 mol) Produkt 34b und 1.79 g (0.008 mol) 2,3-Diaminobenzoesäureamid umgesetzt. Es wurden 0.25 g eines braunen Feststoffes erhalten.
   1H-NMR (D₄-Methanol): δ = 3.85-3.95 (4H), 4.35-4.45 (4H), 7.35 (1H), 7.55-7.7 (4H), 7.75-7.85 (2H), 7.95 (1H), 8.05 (1H), 8.2 (1H), 9.05 (1H) ppm.
   MS: m/e = 399 (M⁺+H)

### Beispiel 35:

### 2-(6-(4-Benzyl-piperazin-1-yl)-pyridin-3-yl)-benzimidazol-4-carbonsäureamid × 2 HCl

Die Synthese erfolgte analog Beispiel 34.
1H-NMR (D₆-DMSO): δ = 3.0-3.2 (2H), 3.3-3.45 (2H), 3.5-3.6 (2H), 4.4 (2H), 4.55-4.7 (2H), 7.2 (1H), 7.4-7.5 (4H), 7.7 (2H), 7.75 (1H), 7.8 (1H), 7.85 (1H), 8.5 (1H), 8.8 (breit, 1H), 9.1 (1H), 10.4 (breit, 1H) ppm.
MS: m/e = 412 (M⁺)

### Beispiel 36:

### 2-(6-(4-tert.-Butyl-piperazin-1-yl)-pyridin-3-yl)-benzimidazol-4-carbonsäureamid

Die Synthese erfolgte analog Beispiel 34.
1H-NMR (D₆-DMSO): **δ** = 1.4 (9H), 3.0-3.2 (2H), 3.45-3.7 (4H), 4.5-4.7 (2H), 7.15 (1H). 7.3 (1H), 7.7-7.75 (2H), 7.8 (1H), 8.45 (1H), 9.05 (1H), 9.3 (1H), 10.7 (breit, 1H) ppm.
MS: m/e = 379 (M⁺+H)

### Beispiel 37:

### 2-(6-(4-n-Butyl)-piperazin-1-yl-pyridin-3-yl)-benzimidazol-4-carbonsäureamid × HCl

Die Synthese erfolgte analog Beispiel 34.
1H-NMR (D₆-DMSO): δ = 0.9-1.0 (3H), 1.35 (2H), 1.75 (2H), 3.0-3.2 (4H), 3.5-3.7 (4H), 4.5-4.7 (2H), 7.25 (1H), 7.55 (1H), 7.8-8.0 (3H), 8.5-8.7 (2H), 9.15 (1H), 11.4 (breit, 1H) ppm.
MS: m/e = 378 (M⁺)

### Beispiel 38:

### 2-(6-(Piperidin-1-yl)-pyridin-3-yl)-benzimidazol-4-carbonsäureamid

Die Synthese erfolgte analog Beispiel 34.
MS: m/e = 322 (M⁺+H)

### Beispiel 39:

### 2-(6-(Pyrrolidin-1-yl)-pyridin-3-yl)-benzimidazol-4-carbonsäureamid

Die Synthese erfolgte analog Beispiel 34.
MS: m/e = 308 (M⁺+H)

### Beispiel 40:

### 2-(6-(2-(Pyrrolidin-1-yl)-eth-1-yl-amino)-pyridin-3-yl)-benzimidazol-4-carbonsäureamid

Die Synthese erfolgte analog Beispiel 34.
MS: m/e = 351 (M⁺+H)

### Beispiel 41:

### 2-(6-(Piperazin-1-yl)-pyridin-3-yl)-benzimidazol-4-carbonsäureamid

Die Synthese erfolgte analog Beispiel 34.
MS: m/e = 323 (M⁺+H)

### Beispiel 42:

### 2-(6-(4-Methyl-piperazin-1-yl)-pyridin-3-yl)-benzimidazol-4-carbonsäureamid

Die Synthese erfolgte analog Beispiel 34.
MS: m/e = 337 (M⁺+H)

### Beispiel 43:

### 2-(6-(4-Ethyl-piperazin-1-yl)-pyridin-3-yl)-benzimidazol-4-carbonsäureamid

Die Synthese erfolgte analog Beispiel 34.
MS: m/e = 351 (M⁺+H)

### Beispiel 44:

### 2-(6-(2-(Piperidin-1-yl)-eth-1-yl-amino)-pyridin-3-yl)-benzimidazol-4-carbonsäureamid

Die Synthese erfolgte analog Beispiel 34.
MS: m/e = 365 (M⁺+H)

### Beispiel 45:

### 2-(6-(3-(N,N-Dimethylamino)-prop-1-yl-amino)-pyridin-3-yl)-benzimidazol-4-carbonsäureamid

Die Synthese erfolgte analog Beispiel 34.
MS: m/e = 367 (M⁺+H)

### Beispiel 46:

### 2-(6-(3-(4-Methylpiperazin-1-yl)-prop-1-yl-amino)-pyridin-3-yl)-benzimidazol-4-carbonsäureamid

Die Synthese erfolgte analog Beispiel 34.
MS: m/e = 394 (M⁺+H)

### Beispiel 47:

### 2-(6-(2-(N,N-Diethylamino)-eth-1-yl-amino)-pyridin-3-yl)-benzimidazol-4-carbonsäureamid

Die Synthese erfolgte analog Beispiel 34.
MS: m/e = 353 (M⁺+H)

### Beispiel 48:

### 2-(6-(2-(N,N-Diethylamino)-eth-1-yl-oxy)-pyridin-2-yl)-benzimidazol-4-carbonsäureamid × Fumarsäure

a) 6-(2-(N,N-Diethylamino)-eth-1-yl-oxy)-picolinsäure-tert.butylester
   1.03 g (8.82 mmol) 2-Diethylaminoethanol wurden in 25 ml Dimethylformamid vorgelegt. Bei Raumtemp. wurden 0.22 g (9.26 mmol) Natriumhydrid (60% Suspension in Weißöl) zugegeben. Nach 30 Minuten Rühren bei Raumtemp. wurde eine Lösung von 2.03 g (8.882 mmol) 6-Brom-picolinsäure-tert.-butylester in 20 ml Dimethylformamid zugetropft. Der Reaktionsansatz wurde 16 Stunden bei Raumtemp. gerührt. Nach Zersetzung überschüssigen Natriumhydrids mit Wasser wurde der Reaktionsansatz im Vakuum eingeengt. Der Rückstand wurde durch Flashchromatographie (Toluol/Tetrahydrofuran/Methanol, 4/1/1 + 2.5% Triethylamin) gereinigt. Es wurden 0.67 g eines hellbraunen Öls erhalten.
b) Eine Lösung von 0.55 g (2.07 mmol) Produkt 42a in 7.5 ml Dioxan und 7.5 ml 2M Salzsäure wurde 3 Stunde bei 100°C gerührt.
   Nach Einengen des Reaktionsansatzes im Vakuum und Trocknen des Rückstandes im Vakuum bei 50°C wurden 0.58 g eines gelben Harzes erhalten.
c) 2-(6-(2-(N,N-Diethylamino)-eth-1-yl-oxy)-pyridin-2-yl)-benzimidazol-4-carbonsäureamid × Fumarsäure
   Analog Beispiel 24c wurden 0.49 g (1.78 mmol) Produkt 42b und 0.40 g (1.78 mmol) 2,3-Diaminobenzoesäureamid umgesetzt. Der Rückstand wurde in Isopropanol aufgenommen und mit Fumarsäure das Fumarat gefällt. Es wurden 40 mg eines gelben Pulvers erhalten.
   1H-NMR (D₆-DMSO): δ = 1.1-1.3 (6H), 3.1-3.25 (4H), 3.4-3.5 (2H), 4.85-5.0 (2H), 7.05 (1H), 7.4 (1H), 7.8-8.0 (4H), 8.1 (1H), 9.3 (breit, 1H), 13.5 (breit, 1H) ppm.
   MS: m/e = 354 (M⁺+H)

### Beispiel 49:

### 2-(6-((2-(Piperidin-1-yl)-eth-1-yl)-oxy)-pyridin-2-yl)-benzimidazol-4-carbonsäureamid

a) 6-((2-(Piperidin-1-yl)-eth-1-yl)-oxy)-picolinsäureethylester × HCl
   Eine Mischung von 1.50 g (9 mmol) 6-Hydroxypicolinsäureethylester, 1.33 g (9 mmol) 2-Chlorethylpiperidin, 2.49 g (18 mmol) Kaliumcarbonat und eine Spatelspitze 18-Krone-6 in 25 ml Dimethylformamid wurde 6 Stunden bei 120°C gerührt. Nach Abtrennen der Feststoffe wurde der Reaktionsansatz im Vakuum eingeengt. Der Rückstand wurde in Ethylacetat aufgenommen und bis zur Trübung mit 1M etherischer Salzsäure-Lösung versetzt. Mit Diethylether wurde das Hydrochlorid gefällt. Nach Filtration und Waschen mit Tetrahydrofuran wurde der Rückstand bei 35°C im Vakuum getrocknet. Es wurden 1.97 g weiße Kristalle erhalten.
b) 6-((2-(Piperidin-1-yl)-eth-1-yl)-oxy)-picolinsäure
   1.77 g (5.62 mmol) Produkt 43a wurden in 25 ml Ethanol mit 25 ml 2M Natronlauge bei Raumtemp. über Nacht gerührt. Der Reaktionsansatz wurde mit 25 ml 2M Salzsäure versetzt und anschließend im Vakuum eingeengt. Der Rückstand wurde mit Dimethylformamid ausgezogen, und das Filtrat wurde im Vakuum eingeengt Der Rückstand wurde in wenig Ethanol aufgenommen, und mit: Diethylether wurde das Produkt gefällt. Es wurden 1.13 g eines beigefarbenen Pulvers erhalten.
c) 2-(6-((2-(Piperidin-1-yl)-eth-1-yl)-oxy)-pyridin-2-yl)-benzimidazol-4-carbonsäureamid × ½ Fumarsäure
   Analog Beispiel 24c wurden 1.08 g (4.31 mmol) Produkt 43b und 0.97 g (4.31 mmol) 2,3-Diaminobenzoesäureamid umgesetzt. Die Reinigung erfolgte über Flashchromatographie (Toluol/Tetrahydrofuran/Methanol, 4/1/1 + 2.5% Triethylamin). Das so erhaltene Rohprodukt wurde in Isopropanol aufgenommen, und mit Fumarsäure wurde das Hemifumarat gefällt. Es wurden 0.71 g beigefarbene Kristalle erhalten.
   1H-NMR (D₆-DMSO): δ = 1.35-1.45 (2H), 1.45-1.6 (4H), 1.55-1.75 (4H), 1.85-1.95 (2H), 4.6-4.75 (2H), 6.6 (1H), 7.0 (1H), 7.4 (1H), 7.8-7.95 (4H), 8.05 (1H), 9.3 (1H) ppm.
   MS: m/e = 366 (M⁺+H)

### Beispiel 50:

### 2-(6-((3-(N-Benzyl-, N-methyl-amino)-prop-1-yl)-oxy)-pyridin-2-yl)-benzimidazol-4-carbonsäureamid

Die Synthese erfolgte analog Beispiel 49.
1H-NMR (D₆-DMSO): **δ** = 2.3-2.4 (2H), 2.7 (3H), 4.3-4.5 (4H), 4.6-4.7 (2H), 7.0 (1H), 7.35-7.45 (4H), 7.55-7.65 (2H), 7.8-8.0 (4H), 8.1-8.15 (1H), 9.1 (1H), 11.0 (1H) ppm.
MS: m/e = 416 (M⁺+H)

### Beispiel 51:

### 2-(6-((3-(N,N-Diethylamino)-prop-1-yl)-oxy)-pyridin-2-yl)-benzimidazol-4-carbonsäureamid

Die Synthese erfolgte analog Beispiel 49.
1H-NMR (D₆-DMSO): δ = 1.2-1.3 (6H), 2.15-2.25 (2H), 3.1-3.4 (6H), 4.6-4.7 (2H), 7.0 (1H), 7.4 (1H). 7.8-8.0 (4H), 8.1 (1H), 9.2 (1H), 10.0 (breit, 1H) ppm.
MS: m/e = 368 (M⁺+H)

### Beispiel 52:

### 2-(6-((3-(4-Methyl-piperazin-1-yl)-prop-1-yl)-oxy)-pyridin-2-yl)-benzimidazol-4-carbonsäureamid × 2 Fumarsäure

Die Synthese erfolgte analog Beispiel 49.
1H-NMR (D₆-DMSO): δ = 1.9-2.0 (2H), 2.25 (3H), 3.3-3.6 (10H), 4.5-4.6 (2H), 6.6 (4H), 7.0 (1H), 7.45 (1H), 7.8 (1H), 7.9-7.95 (2H), 8.0 (1H), 9.3 (1H) ppm.
MS: m/e = 395 (M⁺+H)

### Beispiel 53:

### 2-(6-((3-(N,N-Diethylamino)-prop-1-yl)-oxy)-pyridin-3-yl)-benzimidazol-4-carbonsäureamid × HCl

Die Synthese erfolgte analog Beispiel 49.
1H-NMR (D₆-DMSO): δ = 1.2-1.3 (6H), 2.1-2.25 (2H), 3.1-3.25 (6H), 4.4-4.5 (2H), 7.1 (1H), 7.4 (1H), 7.7-7.8 (2H), 7.9 (1H). 8.6 (1H), 9.05 (1H), 9.1 (1H), 10.3 (1H) ppm.
MS: m/e = 367 (M⁺+H)

### Beispiel 54:

### 2-(6-(Benzyl-oxy)-pyridin-3-yl)-benzimidazol-4-carbonsäureamid

Die Synthese erfolgte analog Beispiel 49.
MS: m/e = 345 (M⁺+H)

### Beispiel 55:

### 2-(6-((3-(N-Benzyl-, N-methyl-amino)-prop-1-yl)-oxy)-pyridin-3-yl)-benzimidazol-4-carbonsäureamid

Die Synthese erfolgte analog Beispiel 49.
MS: m/e = 416 (M⁺+H)

### Beispiel 56:

### 2-(6-((3-(4-Methyl-piperazin-1-yl)-prop-1-yl)-oxy)-pyridin-3-yl)-benzimidazol-4-carbonsäureamid

Die Synthese erfolgte analog Beispiel 49.
MS: m/e = 395 (M⁺+H)

### Beispiel 57:

### 2-(6-((2-Piperidin-eth-1-yl)-oxy)-pyridin-3-yl)-benzimidazol-4-carbonsäureamid

Die Synthese erfolgte analog Beispiel 49.
MS: m/e = 366 (M⁺+H)

### Beispiel 58:

### 2-(6-((2-(N,N-Diethylamino)-eth-2-yl)-oxy)-pyridin-3-yl)-benzimidazol-4-carbonsäureamid

Die Synthese erfolgte analog Beispiel 49.
MS: m/e = 254 (M⁺+H)

### Beispiel 59:

### 2-(6-(4-Benzylamino-phenyl-oxy)-pyridin-3-yl)-benzimidazol-4-carbonsäureamid

Die Synthese erfolgte analog Beispiel 49.
MS: m/e = 436 (M⁺+H)

Nach der folgenden allgemeinen Vorschrift.wurde die Synthese der Beispiele 60-133 in automatisierter Parallelsynthese durchgeführt:
Aldehyd (0.2 mmol) und 2,3-Diaminobenzoesäureamid (0.2 mmol) wurden zusammen mit Na₂S₂O₅ (0.26 mmol) in 5 ml Dimethylformamid vorgelegt und 2h bei 140°C gerührt. Nach dem Entfernen des Lösungsmittels im Vakuum wurde der Rückstand in Dichlormethan aufgenommen und mit Wasser und 1 N wässriger HCl gewaschen. Die wässrige HCl-Phase wurde mit gesättigter Natriumhydrogencarbonat-Lösung neutralisiert und mit Dichlormethan extrahiert. Die organische Phase wurde im Vakuum eingeengt, und das Rohprodukt wurde anschließend chromatographisch gereinigt.

### Beispiel 60:

### 2-(2-(4-Methyl-phenyl)-oxazol-4-yl)-benzimidazal-4-carbonsäureamid

MS: m/e = 319 (M⁺+H)

### Beispiel 61:

### 2-(1-(4-Fluor-phenyl)-5-methyl-pyrazol-4-yl)-benzimidazol-4-carbonsäureamid

MS: m/e = 336 (M⁺+H)

### Beispiel 62:

### 2-(1-(4-Chlor-phenyl)-pyrazol-5-yl)-benzimidazol-4-carbonsäureamid

MS: m/e = 339 (M⁺+H)

### Beispiel 63:

### 2-(2-(4-Chlor-phenyl)-oxazol-4-yl)-benzimidazol-4-carbonsäureamid

MS: m/e = 340 (M⁺+H)

### Beispiel 64:

### 2-(3-Propyl-isoxazol-5-yl)-benzimidazol-4-carbonsäureamid

MS: m/e = 271 (M⁺+H)

### Beispiel 65:

### 2-(3-Ethyloxycarbonyl-pyrazol-5-yl)-benzimidazol-4-carbonsäureamid

MS: m/e = 300 (M⁺+H)

### Beispiel 66:

### 2-(1-(4-Bromphenyl)-pyrrol-3-yl)-benzimidazol-4-carbonsäureamid

MS: m/e = 382 (M⁺+H)

### Beispiel 67:

### 2-((5-Acetyloxymethyl)-furan-2-yl)-benzimidazol-4-carbonsäureamid

MS: m/e = 300 (M⁺+H)

### Beispiel 68:

### 2-(N-Methylindol-3-yl)-benzimidazol-4-carbonsäureamid

MS: m/e = 291 (M⁺+H)

### Beispiel 69:

### 2-Pyrrol-2-yl-benzimidazol-4-carbonsäureamid

MS: m/e = 227 (M⁺+H)

### Beispiel 70:

### 2-(2-Methyl-5-nitro-indol-3-yl)-benzimidazol-4-carbonsäureamid

MS: m/e = 338 (M⁺+H)

### Beispiel 71:

### 2-(N-Acetyl-indol-3-yl)-benzimidazol-4-carbonsäureamid

MS: m/e = 319 (M⁺+H)

### Beispiel 72:

### 2-(5-Chlor-indol-3-yl)-benzimidazol-4-carbonsäureamid

MS: m/e = 311 (M⁺+H)

### Beispiel 73:

### 2-(1-(4-Methoxy-phenyl)-pyrrol-3-yl)-benzimidazol-4-carbonsäureamid

MS: m/e = 333 (M⁺+H)

### Beispiel 74:

### 2-(1,2,5-Trimethyl-pyrrol-3-yl)-benzimidazol-4-carbonsäureamid

MS: m/e = 269 (M⁺+H)

### Beispiel 75:

### 2-(2-Methyl-indol-3-yl)-benzimidazol-4-carbonsäureamid

MS: m/e = 291 (M⁺+H)

### Beispiel 76:

### 2-(3-Phenyl-pyrazol-4-yl)-benzimidazol-4-carbonsäureamid

MS: m/e = 304 (M⁺+H)

### Beispiel 77:

### 2-(6-Methoxycarbonyl-indol-3-yl)-benzimidazol-4-carbonsäureamid

MS: m/e = 335 (M⁺+H)

### Beispiel 78:

### 2-(2-Ethyl-imidazol-4-yl)-benzimidazol-4-carbonsäureamid

MS: m/e = 255 (M⁺+H)

### Beispiel 79:

### 2-(4-(2,6-Difluorophenyl-1-carbonyl)-1-methyl-pyrrol-2-yl)-benzimidazol-4-carbonsäureamid

MS: m/e = 381 (M⁺+H)

### Beispiel 80:

### 2-(4-(4-Fluorophenyl-1-carbonyl)-1-methyl-pyrrol-2-yl)-benzimidazol-4-carbonsäureamid

MS: m/e = 363 (M⁺+H)

### Beispiel 81:

### 2-(1-Methyl-4-(phenyl-1-carbonyl)-pyrrol-2-yl)-benzimidazol-4-carbonsäureamid

MS: m/e = 345 (M⁺+H)

### Beispiel 82:

### 2-(1-(4-Chlor-phenyl)-pyrrol-3-yl)-benzimidazol-4-carbonsäureamid MS: m/e = 338 (M⁺+H)

### Beispiel 83:

### 2-(2-(2,4-Dichlor-phenyl)-oxazol-4-yl)-benzimidazol-4-carbonsäureamid

MS: m/e = 374 (M⁺+H)

### Beispiel 84:

### 2-(1-(2,4-Dichlorophenyl)-5-methyl-pyrazol-4-yl)-benzimidazol-4-carbonsäureamid

MS: m/e = 387 (M⁺+H)

### Beispiel 85:

### 2-(2,5-Dibrom-thiophen-3-yl)-benzimidazol-4-carbonsäureamid

MS: m/e = 402 (M⁺+H)

### Beispiel 86:

### 2-(2-Phenyl-oxazol-4-yl)-benzimidazol-4-carbonsäureamid

MS: m/e = 305 (M⁺+H)

### Beispiel 87:

### 2-(3-Hydroxy-5-hydroxyethyl-2-methyl-pyridin-4-yl)-benzimidazol-4-carbonsäureamid

MS: m/e = 299 (M⁺+H)

### Beispiel 88:

### 2-(1-(2,3,4-Trichlor-phenyl)-pyrrol-3-yl)-benzimidazol-4-carbonsäureamid

MS: m/e = 407 (M⁺+H)

### Beispiel 89:

### 2-(Indol-3-yl)-benzimidazol-4-carbonsäureamid

MS: m/e = 277 (M⁺+_{H})

### Beispiel 90:

### 2-(1-(4-Chlor-2-nitro-phenyl)-pyrrol-3-yl)-benzimidazol-4-carbonsäureamid

MS: m/e = 385 (M⁺+H)

### Beispiel 91:

### 2-(6-Methyl-pyridin-2-yl)-benzimidazol-4-carbonsäureamid

MS: m/e = 253 (M⁺+H)

### Beispiel 92:

### 2-(1-(Benzylamino-carbonyl-methyl)-pyrrol-2-yl)-benzimidazol-4-carbonsäureamid

MS: m/e = 374 (M⁺+H)

### Beispiel 93:

### 2-(4-Methyl-5-(4-trifluormethyl-phenyl)-isoxazol-3-yl)-benzimidazol-4-carbonsäureamid

MS: m/e = 387 (M⁺+H)

### Beispiel 94:

### 2-(1-Phenyl-pyrazol-4-yl)-benzimidazol-4-carbonsäureamid

MS: m/e = 304 (M⁺+H)

### Beispiel 95:

### 2-(1-(4-Chlor-phenyl)-pyrazol-4-yl)-benzimidazol-4-carbonsäureamid

MS: m/e = 339 (M⁺+H)

### Beispiel 96:

### 2-(5-Methyl-1-phenyl-pyrazol-4-yl)-benzimidazol-4-carbonsäureamid

MS: m/e = 318 (M⁺+H)

### Beispiel 97:

### 2-(1-(3-Cyano-4-methoxy-pyridin-2-yl)-pyrrol-2-yl)-benzimidazol-4-carbonsäureamid

MS: m/e = 359 (M⁺+H)

### Beispiel 98:

### 2-(1-(4-Tolylsulfonyl)-indol-3-yl)-benzimidazol-4-carbonsäureamid

MS: m/e = 431 (M⁺+H)

### Beispiel 99:

### 2-(5-Methoxy-indol-3-yl)-benzimidazol-4-carbonsäureamid

MS: m/e = 307 (M⁺+H)

### Beispiel 100:

### 2-(2-Phenyl-imidazol-4-yl)-benzimidazol-4-carbonsäureamid

MS: m/e = 304 (M⁺+H)

### Beispiel 101:

### 2-(1-(2-nitro-phenyl-sulfonyl)-pyrrol-2-yl)-benzimidazol-4-carbonsäureamid

MS: m/e = 412 (M⁺+H)

### Beispiel 102:

### 2-(4-Brom-1-(4-chlor-phenyl-methyl)-pyrazol-5-yl)-benzimidazol-4-carbonsäureamid

MS: m/e = 431 (M⁺+H)

### Beispiel 103:

### 2-(2-(4-Fluor-phenyl-carbonyl)-benzofuran-5-yl)-benzimidazol-4-carbonsäureamid

MS: m/e = 400 (M⁺+H)

### Beispiel 104:

### 2-(1-(2,4-Difluor-phenyl-sulfonyl)-pyrrol-2-yl)-benzimidazol-4-carbonsäureamid

MS: m/e = 403 (M⁺+H)

### Beispiel 105:

### 2-(1-(4-Methyl-phenyl)-pyrrol-2-yl)-benzimidazol-4-carbonsäureamid

MS: m/e = 317 (M⁺+H)

### Beispiel 106:

### 2-(4-(4-Chlor-phenyl-carbonyl)-1-methyl-pyrrol-2-yl)-benzimidazol-4-carbonsäureamid

MS: m/e = 380 (M⁺+H)

### Beispiel 107:

### 2-(2-(4-Fluor-phenyl)-indol-3-yl)-benzimidazol-4-carbonsäureamid MS: m/e = 371 (M⁺+H)

### Beispiel 108:

### 2-(3,7-Dichlor-chinolin-8-yl)-benzimidazol-4-carbonsäureamid

MS: m/e = 358 (M⁺+H)

### Beispiel 109:

### 2-(5-Chlor-3-methyl-1-phenyl-pyrazol-4-yl)-benzimidazol-4-carbonsäureamid

MS: m/e = 353 (M⁺+H)

### Beispiel 110:

### 2-(5-Methyl-furan-2-yl)-benzimidazol-4-carbonsäureamid

MS: m/e = 242 (M⁺+H)

### Beispiel 111:

### 2-(1-(2-Chlor-phenyl)-5-trifluormethyl-pyrazol-4-yl)-benzimidazol-4-carbonsäureamid

MS: m/e = 407 (M⁺+H)

### Beispiel 112:

### 2-(1-(2,4-Dichlor-phenyl)-5-trifluormethyl-pyrazol-4-yl)-benzimidazol-4-carbonsäureamid

MS: m/e = 441 (M⁺+H)

### Beispiel 113:

### 2-(1-tert.-Butyl-pyrazol-4-yl)-benzimidazol-4-carbonsäureamid

MS: m/e = 284 (M⁺+H)

### Beispiel 114:

### 2-(5-Methyl-imidazol-4-yl)-benzimidazol-4-carbonsäureamid

MS: m/e = 242 (M⁺+H)

### Beispiel 115:

### 2-(4-Chlor-5-nitro-benzothiophen-2-yl)-benzimidazol-4-carbonsäureamid

MS: m/e = 374 (M⁺+H)

### Beispiel 116:

### 2-(1-Dimethylamino-3-methoxy-isochinolin-4-yl)-benzimidazol-4-carbonsäureamid

MS: m/e = 362 (M⁺+H)

### Beispiel 117:

### 2-(1-Phthalimido-butyl-indol-3-yl)-benzimidazol-4-carbonsäureamid

MS: m/e = 478 (M⁺+H)

### Beispiel 118:

### 2-(1-Methyl-pyrazol-4-yl)-benzimidazol-4-carbonsäureamid

MS: m/e = 242 (M⁺+H)

### Beispiel 119:

### 2-(1-(2,6-Dimethyl-phenyl)-pyrrol-3-yl)-benzimidazol-4-carbonsäureamid

MS: m/e = 331 (M⁺+H)

### Beispiel 120:

### 2-(2-Dimethylamino-thiazol-5-yl-)-benzimidazol-4-carbonsäureamid

MS: m/e = 288 (M⁺+H)

### Beispiel 121:

### 2-(1-tert.-Butyl-pyrrol-3-yl)-benzimidazol-4-carbonsäureamid

MS: m/e = 283 (M⁺+H)

### Beispiel 122:

### 2-(3-but-2-yl-isoxazol-5-yl)-benzimidazol-4-carbonsäureamid

MS: m/e = 285 (M⁺+H)

### Beispiel 123:

### 2-(3-iso-butyl-isoxazol-5-yl)-benzimidazol-4-carbonsäureamid

MS: m/e = 284 (M⁺+H)

### Beispiel 124:

### 2-(3-(4-tert.-Butyl-phenyl)-isoxazol-5-yl)-benzimidazol-4-carbonsäureamid

MS: m/e = 361 (M⁺+H)

### Beispiel 125:

### 2-(3-tert.-Butyl-isoxazol-5-yl)-benzimidazol-4-carbonsäureamid

MS: m/e = 285 (M⁺+H)

### Beispiel 126:

### 2-(3-Phenyl-isoxazol-5-yl)-benzimidazol-4-carbonsäureamid

MS: m/e = 305 (M⁺+H)

### Beispiel 127:

### 2-(3-tert.-Butyl-5-phenyl-isoxazol-4-yl)-benzimidazol-4-carbonsäureamid

MS: m/e = 361 (M⁺+H)

### Beispiel 128:

### 2-(1-(4-Chlor-phenyl)-5-methyl-pyrazol-4-yl)-benzimidazol-4-carbonsäureamid

MS: m/e = 353 (M⁺+H)

### Beispiel 129:

### 2-(1-(4-Chlor-phenyl)-3-methyl-pyrazol-4-yl)-benzimidazol-4-carbonsäureamid

MS: m/e = 353 (M⁺+H)

### Beispiel 130:

### 2-(1-(4-Brom-phenyl)-pyrazol-4-yl)-benzimidazol-4-carbonsäureamid

MS: m/e = 383 (M⁺+H)

### Beispiel 131:

### 2-(1-(4-Chlor-phenyl)-3,5-dimethyl-pyrazol-4-yl)-benzimidazol-4-carbonsäureamid

MS: m/e = 366 (M⁺+H)

### Beispiel 132:

### 2-(1-(4-Methoxy-phenyl)-5-trifluormethyl-pyrazol-4-yl)-benzimidazol-4-carbonsäureamid

MS: m/e = 402 (M⁺+H)

### Beispiel 133:

### 2-(4-Methyl-5-phenyl-isoxazol-3-yl)-benzimidazol-4-carbonsäureamid

MS: m/e = 319 (M⁺+H)

Folgende erfindungsgemäße Verbindungen können analog den oben beschriebenen Methoden hergestellt werden:
1. 2-Pyridin-2-yl-benzimidazol-4-carbonsäureamid
2. 2-(2-(2(N,N-Dimethylamino)-eth-1-yl-amino)-pyridin-3-yl)-benzimidazol-4-carbonsäureamid
3. 2-(2-(2-Pyrrolidin-1-yl-eth-1-yl-amino)-pyridin-3-yl)-benzimidazol-4-carbonsäureamid
4. 2-(2-(2-Piperidin-1-yl-eth-1-yl-amino)-pyridin-3-yl)-benzimidazol-4-carbonsäureamid
5. 2-(2-(2-Homopiperidin-1-yleth-1-yl-amino)-pyridin-3-yl)-benzimidazol-4-carbonsäureamid
6. 2-(2-(2-(4-Phenyl-piperidin-1-yl)-eth-1-yl-amino)-pyridin-3-yl)-benzimidazol-4-carbonsäureamid
7. 2-(2-(2-Piperazin-1-yl-eth-1-yl-amino)-pyridin-3-yl)-benzimidazol-4-carbonsäureamid
8. 2-(2-(2-(4-Methylpiperazin-1-yl)-eth-1-yl-amino)-pyridin-3-yl)-benzimidazol-4-carbonsäureamid
9. 2-(2-(2-(4-Benzylpiperazin-1-yl)-eth-1-yl-amino)-pyridin-3-yl)-benzimidazol-4-carbonsäureamid
10. 2-(2-(2 (N,N-Dimethylamino)-eth-1-yl)-methylamino)-pyridin-3-yl)-benzimidazol-4-carbonsäureamid
11. 2-(2-(2-Pyrrolidin-1-yl-eth-1-yl)-methylamino)-pyridin-3-yl)-benzimidazol-4-carbonsäureamid
12. 2-(2-(2-Piperidin-1-yl-eth-1-yl)-methylamino)-pyridin-3-yl)-benzimidazol-4-carbonsäureamid
13. 2-(2-(2-Homopiperidin-1-yl-eth-1-yl)-methylamino)-pyridin-3-yl)-benzimidazol-4-carbonsäureamid
14. 2-(2-(2 (4-Phenyl-piperidin-1-yl)-eth-1-yl)-methylamino)-pyridin-3-yl)-benzimidazol-4-carbonsäureamid
15. 2-(2-(2-Piperazin-1-yl-eth-1-yl)-methylamino)-pyridin-3-yl)-benzimidazol-4-carbonsäureamid
16. 2-(2-(2(4-Methylpiperazin-1-yl)-eth-1-yl)-methylamino)-pyridin-3-yl)-benzimidazol-4-carbonsäureamid
17. 2-(2-(2-(4-Benzylpiperazin-1-yl)-eth-1-yl)-methylamino)-pyridin-3-yl)-benzimidazol-4-carbonsäureamid
18. 2-(2-(3-(N,N-Dimethylamino)-prop-1-yl-amino)-pyridin-3-yl)-benzimidazol-4-carbonsäureamid
19. 2-(2-(3-Pyrrolidin-1-yl-prop-1-yl-amino)-pyridin-3-yl)-benzimidazol-4-carbonsäureamid
20. 2-(2-(3-Piperidin-1-yl-prop-1-yl-amino)-pyridin-3-yl)-benzimidazol-4-carbonsäureamid
21. 2-(2-(3-Homopiperidin-1-yl-prop-1-yl-amino)-pyridin-3-yl)-benzimidazol-4-carbonsäureamid
22. 2-(2-(3-(4-Phenyl-piperidin-1-yl)-prop-1-yl-amino)-pyridin-3-yl)-benzimidazol-4-carbonsäureamid
23. 2-(2-(3-Piperazin-1-yl-prop-1-yl-amino)-pyridin-3-yl)-benzimidazol-4-carbonsäureamid
24. 2-(2-(3-(4-Methylpiperazin-1-yl)-prop-1-yl-amino)-pyridin-3-yl)-benzimidazol-4-carbonsäureamid
25. 2-(2-(3-(4-Benzylpiperazin-1-yl)-prop-1-yl-amino)-pyridin-3-yl)-benzimidazol-4-carbonsäureamid
26. 2-Chinoxalin-2-yl-benzimidazol-4-carbonsäureamid
27. 2-Benzofuran-2-yl-benzimidazol-4-carbonsäureamid
28. 2-Benzotriazol-5-yl-benzimidazol-4-carbonsäureamid
29. 2-Thiazol-2-yl-benzimidazol-4-carbonsäureamid
30. 2-Pyridazin-4-yl-benzimidazol-4-carbonsäureamid
31. 2(1(2(N,N-Diethylamino)-eth-1-yl)-pyrrol-2-yl)-benzimidazol-4-carbonsäureamid
32. 2-(4-Hydroxypyridin-3-yl)-benzimidazol-4-carbonsäureamid
33. 2-(4-Methoxypyridin-3-yl)-benzimidazol-4-carbonsäureamid
34. 2-(4-Benzyloxypyridin-3-yl)-benzimidazol-4-carbonsäureamid
35. 2-(4-(2(N,N-Dimethylamino)-eth-1-yl-oxy)pyridin-3-yl)-benzimidazol-4-carbonsäureamid ;
36. 2-(4-(2(N,N-Diethylamino)-eth-1-yl-oxy)-pyridin-3-yl)-benzimidazol-4-carbonsäureamid
37. 2-(4-(3(N,N-Dimethylamino)-prop-1-yl-oxy)-pyridin-3-yl)-benzimidazol-4-carbonsäureamid
38. 2-(4-(3(N,N-Diethylamino)-prop-1-yl-oxy)-pyridin-3-yl)-benzimidazol-4-carbonsäureamid
39. 2-(4-(2-Pyrrolidin-1-yl-eth-1-yl-oxy)-pyridin-3-yl)-benzimidazol-4-carbonsäureamid
40. 2-(4-(3-Pyrrolidin-1-yl-prop-1-yl-oxy)-pyridin-3-yl)-benzimidazol-4-carbonsäureamid
41. 2-(4-(2-Piperidin-1-yl-eth-1-yl-oxy)-pyridin-3-yl)-benzimidazol-4-carbonsäureamid
42. 2-(4-(3-Piperidin-1-yl-prop-1-yl-oxy)-pyridin-3-yl)-benzimidazol-4-carbonsäureamid
43. 2-(4-(2-Piperazin-1-yl-eth-1-yl-oxy)-pyridin-3-yl)-benzimidazol-9-carbonsäureamid
44. 2-(4-(3-Piperazin-1-yl-prop-1-yl-oxy)-pyridin-3-yl)-benzimidazol-4-carbonsäureamid
45. 2-(4-(2-(4-Methylpiperazin-1-yl)-eth-1-yl-oxy)pyridin-3-yl)-benzimidazol-4-carbonsäureamid
46. 2-(4-(3-(4-Methylpiperazin-1-yl)-prop-1-yl-oxy)pyridin-3-yl)-benzimidazol-4-carbonsäureamid
47. 2-(4-(2-(4-Benzylpiperazin-1-yl)-eth-1-yl-oxy)pyridin-3-yl)-benzimidazol-4-carbonsäureamid
48. 2-(4-(3-(4-Benzylpiperazin-1-yl)-prop-1-yl-oxy)pyridin-3-yl)-benzimidazol-4-carbonsäureamid
49. 2-(4-(4-Methylpiperazin-1-yl)-pyridin-3-yl)-benzimidazol-4carbonsäureamid
50. 2-(4-(4-Benzylpiperazin-1-yl)-pyridin-3-yl)-benzimidazol-4-carbonsäureamid
51. 2-(4-(4-Ethylpiperazin-1-yl)-pyridin-3-yl)-benzimidazol-4-carbonsäureamid
52. 2-(4-(4-Butylpiperazin-1-yl)-pyridin-3-yl)-benzimidazol-4-carbonsäureamid
53. 2-(6-(3-(N.N-Dimethylamino)-prop-1-yl-amino)-pyridin-3-yl)-benzimidazol-4-carbonsäureamid
54. 2-(6-((3-(N,N-Dimethylamino)-prop-1-yl)-methylamino)-pyridin-3-yl)-benzimidazol-4-carbonsäureamid
55. 2-(6-(2-(N,N-Dimethylamino)-eth-1-yl-amino)-pyridin-3-yl)-benzimidazol-4-carbonsäureamid
56. 2-(6-((2-(N,N-Dimethylamino)-eth-1-yl)-methylamino)-pyridin-3-yl)-benzimidazol-4-carbonsäureamid
57. 2-(6-(3-(Piperazin-1-yl)-prop-1-yl-amino)-pyridin-3-yl)-benzimidazol-4-carbonsäureamid
58. 2-(6-(3-(4-Ethyl-piperazin-1-yl)-prop-1-yl-amino)-pyridin-3-yl)-benzimidazol-4-carbonsäureamid
59. 2-(6-(3-(4-Benzyl-piperazin-1-yl)-prop-1-yl-amino)-pyridin-3-yl)-benzimidazol-4-carbonsäureamid
60. 2-(6-((3-(Piperazin-1-yl)-prop-1-yl)-methylamino)-pyridin-3-yl)-benzimidazol-4-carbonsäureamid
61. 2-(6-((3-(4-Ethyl-piperazin-1-yl)-prop-1-yl)-methylamino)-pyridin-3-yl)-benzimidazol-4-carbonsäureamid
62. 2-(6-((3-(4-Benzyl-piperazin-1-yl)-prop-1-yl)-methylamino)-pyridin-3-yl)-benzimidazol-4-carbonsäureamid
63. 2-(6-(3-(Homopiperazin-1-yl)-prop-1-yl-amino)-pyridin-3-yl)-benzimidazol-4-carbonsäureamid
64. 2-(6-(3-(4-Methyl-homopiperazin-1-yl)-prop-1-yl-amino)-pyridin-3-yl)-benzimidazol-4-carbonsäureamid
65. 2-(6-(3-(9-Ethyl-homopiperazin-1-yl)-prop-1-yl-amino)-pyridin-3-yl)-benzimidazol-4-carbonsäureamid
66. 2-(6-(3-(4-Benzyl-homopiperazin-2-yl)-prop-1-yl-amino)-pyridin-3-yl)-benzimidazol-4-carbonsäureamid
67. 2-(6-((3-(Homopiperazin-1-yl)-prop-1-yl)-methylamino)-pyridin-3-yl)-benzimidazol-4-carbonsäureamid
68. 2-(6-((3-(4-Methyl-homopiperazin-1-yl)-prop-1-yl)-methylamino)-pyridin-3-yl)-benzimidazol-4-carbonsäureamid
69. 2-(6-((3-(4-Ethyl-homopiperazin-1-yl)-prop-1-yl)-methylamino)-pyridin-3-yl)-benzimidazol-4-carbonsäureamid
70. 2-(6-((3-(4-Benzyl-homopiperazin-1-yl)-prop-1-yl)-methylamino)-pyridin-3-yl)-benzimidazol-4-carbonsäureamid
71. 2-(6-(2-(Piperazin-1-yl)-eth-1-yl-amino)-pyridin-3-yl)-benzimidazol-4-carbonsäureamid
72. 2-(6-(2-(4-Methyl-piperazin-1-yl)-eth-1-yl-amino)-pyridin-3-yl)-benzimidazol-4-carbonsäureamid
73. 2-(6-(2-(4-Ethyl-piperazin-1-yl)-eth-1-yl-amino)-pyridin-3-yl)-benzimidazol-4-carbonsäureamid
74. 2-(6-(2-(4-Benzyl-piperazin-1-yl)-eth-1-yl-amino)-pyridin-3-yl)-benzimidazol-4-carbonsäureamid
75. 2-(6-((2-(Piperazin-1-yl)-eth-1-yl)-methylamino)-pyridin-3-yl)-benzimidazol-4-carbonsäureamid
76. 2-(6-((2-(Methyl-piperazin-1-yl)-eth-1-yl)-methylamino)-pyridin-3-yl)-benzimidazol-4-carbonsäureamid
77. 2-(6-((2-(4-Ethyl-piperazin-1-yl)-eth-1-yl)-methylamino)-pyridin-3-y3)-benzimidazol-4-carbonsäureamid
78. 2-(6-((2-(4-Benzyl-piperazin-1-yl)-eth-1-yl)-methylamino)-pyridin-3-yl)-benzimidazol-4-carbonsäureamid
79. 2- (6- (2- (Homopiperazin-1-yl) -eth-1-yl-amino) -pyridin-3-yl)-benzimidazol-4-carbonsäureamid
80. 2-(6-(2-(4-Methyl-homopiperazin-1-yl)-eth-1-yl-amino)-pyridin-3-yl)-benzimidazol-4-carbonsäureamid
81. 2-(6-(2-(4-Ethyl-homopiperazin-1-yl)-eth-1-yl-amino)-pyridin-3-yl)-benzimidazol-4-carbonsäureamid
82. 2-(6-(2-(4-Benzyl-homopiperazin-1-yl)-eth-1-yl-amino)-pyridin-3-yl)-benzimidazol-4-carbonsäureamid
83. 2-(6-((2 -(Homopiperazin-1-yl)-eth-1-yl)-methylamino)-pyridin-3-yl)-benzimidazol-4-carbonsäureamid
84. 2-(6-((2-(4-Methy2-homopiperazin-1-yl)-eth-1-yl)-methylamino)-pyridin-3-yl)-benzimidazol-4-carbonsäureamid
85. 2-(6-((2-(4-Ethyl-homopiperazin-1-yl)-eth-1-yl)-methylamino)-pyridin-3-yl)-benzimidazol-4-carbonsäureamid
86. 2-(6-((2-(4-Benzyl-homopiperazin-1-yl)-eth-1-yl)-methylamino)-pyridin-3-yl)-benzimidazol-4-carbonsäureamid
87. 2-(6-(Homopiperazin-1-yl)-pyridin-3-yl)-benzimidazol-4-carbonsäureamid
88. 2-(6-(4-Methyl-homopiperazin-1-yl)-pyridin-3-yl)-benzimidazol-4-carbonsäureamid
89. 2-(6-(4-Ethyl-homopiperazin-1-yl)-pyridin-3-yl)-benzimidazol-4-carbonsäureamid
90. 2-(6-(4-propyl-homopiperazin-1-yl)-pyridin-3-yl )-benzimidazol-4-carbonsäureamid
91. 2-(6-(4-Butyl-homopiperazin-1-yl)-pyridin-3-yl)-benzimidazol-4-carbonsäureamid
92. 2-(6-(4-tert.-Butyl-homopiperazin-3-yl)-pyridin-3-yl)-benzimidazol-4-carbonsäureamid
93. 2-(6-(4-Phenyl-homopiperazin-1-yl)-pyridin-3-yl)-benzimidazol-4-carbonsäureamid
94. 2-(6-(4-Benzyl-homopiperazin-1-yl)-pyridin-3-yl)-benzimidazol-4-carbonsäureamid
95. 2-(6-(Homopiperidin-1-yl)-pyridin-3-yl)-benzimidazol-4-carbonsäureamid
96. 2-(6-(Morpholin-4-yl)-pyridin-3-yl)-benzimidazol-4-carbonsäureamid

## Patentansprüche

1. Verbindung der Formel I oder II 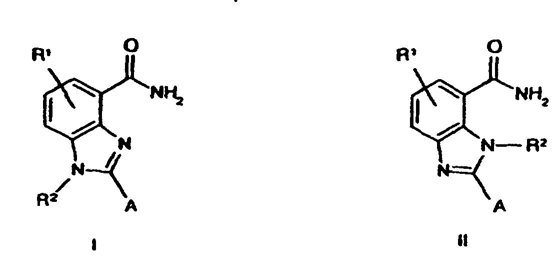 worin
A aromatischer Heteromonocyclus, aromatischer oder teilaromatischer Heterobicyclus und Heterotricyclus bedeutet, wobei die Ringsysteme maximal 15 Kohlenstoffatome und bis zu 4 Heteroatome ausgewählt aus der Gruppe N,O,S enthalten und Cyclen zusätzlich noch bis zu 2 Oxogruppen tragen können und A noch mit bis zu drei unterschiedlichen oder gleichen Resten R³ und zusätzlich einem Rest R⁴ substituiert sein kann und
R¹ Wasserstoff, Chlor, Fluor, Brom, Jod, verzweigtes und unverzweigtes C₁-C₆-Alkyl, OH, Nitro, CF₃, CN, NR¹¹R¹², NH-CO-R¹³, O-C₁-C₄-Alkyl, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeuten, und
R² Wasserstoff, verzweigtes und unverzweigtes C₁-C₆-Alkyl und
R³ Wasserstoff, Chlor, Brom, Iod, Fluor, CF₃, OCF₃, Nitro, NH₂, CO-R⁸, CO₂-R⁸, SO₂-R⁸, OH, O-C₁-C₄-Alkyl, O-C₀-C₄-Alkyl-Phenyl, eine C₁-C₆-Kette, die gesättigt, ungesättigt oder partiell ungesättigt sein kann, und noch mit einem Rest R³³ substituiert sein kann, Phenyl, wobei die Phenyl-Ringe noch mit bis zu drei gleichen oder verschiedenen Resten R³¹ subsitutiert sein können, und Pyridyl, den mit bis zu drei Resten R³² substituiert sein kann, und
R³¹ OH, C₁-C₆-Alkyl, O-C₁-C₄-Alkyl, Chlor, Brom, Jod, Fluor, CF₃, Nitro, NH₂, und
R³² OH, C₁-C₆-Alkyl, O-C₁-C₄-Alkyl, Chlor, Brom, Iod, Fluor, CF₃, Nitro, NH₂, CN und
R³³ CO-NH-R⁸, OH, O-C₁-C₆-Alkyl, O-CO-R⁸ und
R⁴ Wasserstoff und -(D)ₚ-(E)ₛ-(CH₂)_{q} -B bedeutet, wobei
D S, NR⁴³ und O
E Phenyl und
s 0 und 1 und
B NR⁴¹R⁴² und 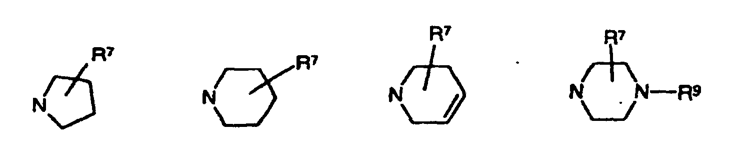 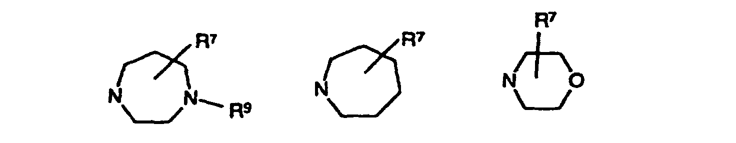
bedeutet und
p 0 und 1 bedeuten kann und
q 0, 1, 2, 3 oder 4 sein kann, und
R⁴¹ Wasserstoff, C₁-C₆-Alkyl, (CH₂)ᵣ-G und
R⁴² Wasserstoff, C₁-C₆-Alkyl, -CO-R⁸, SO₂-R⁸, CO₂-R⁸, -(C=N)-R⁸ und -(C=N)-NHR⁸ und
R⁴¹ und R⁴² einen Phthaloyl-Rest bilden können und
R⁴³ Wasserstoff und C₁-C₄-Alkyl und
r 0.1,2.3,4 und
G Phenyl, der noch maximal zwei Reste R tragen kann, NR¹¹R¹², NH-C₁-C₄-Alkyl-Phenyl, Pyrrolidin, Piperidin, 1,2,5,6-Tetrahydropyridin, Morpholin. Homopiperidin, Piperazin, das noch mit einem Alkyl-Rest C₁-C₆-Alkyl substituiert sein kann, und Homopiperazin, das noch mit einem Alkyl-Rest C₁-C₆-Alkyl substituiert sein kann, und
R⁷ Wasserstoff, C₁-C₆-Alkyl, Phenyl, wobei der Ring noch mit bis zu zwei Resten R⁷¹ substituiert sein kann, und
R⁷¹ OH, C₁-C₆-Alkyl, O-C₁-C₄-Alkyl, Chlor, Brom, Jod, Fluor, CF₃, Nitro, NH₂, und
R⁸ C₁-C₆-Alkyl, CF₃, NR¹¹R¹², Phenyl, C₁-C₄-Alkyl-Phenyl, wobei der Ring noch mit bis zu zwei Resten R⁸¹ substituiert sein kann, und
R⁸¹ OH, C₁-C₆-Alkyl, O-C₁-C₄-Alkyl, Chlor, Brom, Jod, Fluor, CF₃, Nitro, NH₂, und
R⁹ Wasserstoff, CO-R⁸, SO₂-R⁸, CO₂-R⁸, C₁-C₆-Alkyl, C₁-C₄-Alkyl-Phenyl und Phenyl, wobei die Phenyl-Ringe noch mit bis zu zwei Resten R⁹¹ substituiert sein kann, und
R⁹¹ OH, C₁-C₆-Alkyl, O-C₁-C₄-Alkyl, Chlor, Brom, Jod, Fluor, CF₃, Nitro, NH₂, und sein kann.
sowie ihre tautomeren Formen, möglichen enantiomeren und diastereomeren Formen, und deren Prodrugs, ausgewählt aus der Gruppe bestehend aus Phosphaten, Carbamaten von Aminosäuren und Estern.

2. Verbindung der Formel I oder II nach Anspruch 1, wobei
R¹ Wasserstoff und
R² Wasserstoff und C₁-C₄-Alkyl und
D NR⁴³ und O und
p 0 und 1 und
s 0 und
q 0, 1 und 2, wenn p =0 ist, oder q 2 und 3, wenn p = 1 ist, und
R⁴² und R⁴³, unabhängig voneinander, Wasserstoff und C₁-C₄-Alkyl und
R⁷ Wasserstoff und Phenyl und
R⁹ Wasserstoff, C₁-C₄-Alkyl und C₀-C₄-Alkyl-Phenyl sein kann.

3. Verbindungen nach Formel I oder II gemäß einem der Ansprüche 1 oder 2, wobei A folgende Bedeutung hat: Indol, Benzimidazol, Pyrrol, Imidazol, Furan, Thiophen, Benzothiophen, Benzofuran, Pyrazol, Thiazol, Benzothiazol, Phthalimid, Indazol, Benzotriazol, Phthalizin, Indolin, Isoindolin, Pyridin, Chinolin, Pyrimidin, Pyridazin, Isochinolin, Chinoxalin, Chinazolin, Isoxazol, Oxazol, Imidazopyridin, Pyrazin.

4. Verbindungen nach Formel I und II gemäß Anspruch 2, wobei A folgende Bedeutung hat:
Pyridin, Thiophen, Thiazol, Furan, Indol, Oxazol, Pyrazol, Pyrrol, Benzofuran, Imidazol, Benzothiophen, Isoxazol, Pyrazin, Pyrimidin, Pyridazin, Chinolin und der Heterocyclus mit bis zu drei Resten R³ und einem Rest R⁴ substituiert sein kann, wobei
R³ Wasserstoff, Chlor, Brom, Iod, Fluor, COR⁸, CO₂R⁸, SO₂R⁸, C₁-C₄-Alkyl-Phenyl, eine C₁-C₆-Kette, die gesättigt, ungesättigt und partiell ungesättigt sein kann, und noch mit einer Gruppe O-CO-R⁸ substituiert sein kann, Phenyl, wobei die Phenyl-Ringe noch mit bis zu drei gleichen oder verschiedenen Resten R³¹ substituiert sein können, und Pyridyl, das mit bis zu drei gleichen oder verschiedenen Resten R³² substituiert sein kann, und
R⁴ Wasserstoff und (D)ₚ-(E)ₛ-(CH₂)_{q}-B,
und R³ und R⁴ nicht gleichzeitig Wasserstoff sind.

5. Verbindungen nach Formel I und II gemäß Anspruch 2, wobei A folgende Bedeutung hat:
Pyridin, Pyrazin, Pyrimidin, Pyridazin. Chinolin, Thiazol, Thiophen, Pyrrol und Pyrazol und der Heterocyclus mit einem Rest R³ und einem Rest R⁴ substituiert, sein kann, wobei
R³ Wasserstoff, Chlor, Brom, Iod, Fluor, C₁-C₄-Alkyl, und
R⁴ (D)ₚ-(E)ₛ-(CH₂)_{q}-B ist.

6. Verbindungen nach Formel I und II gemäß Anspruch 2, wobei A Pyridin, Thiophen und Thiazol sein kann und der Heterocyclus mit einem Rest R⁴ substituiert ist, wobei R⁴ (D)ₚ-(E)ₛ-(CH₂)_{q}-B ist und R³ Wasserstoff bedeutet.

7. Arzneimittel enthaltend neben üblichen Träger- und Hilfsstoffen Verbindungen nach einem der Ansprüche 1-3.

8. Verwendung von Verbindungen der Formel I bzw. II nach einem der Ansprüche 1 bis 6 zur Herstellung von Arzneimitteln zur Behandlung von neurodegenerativen Krankheiten und neuronalen Schädigungen.

9. Verwendung nach Anspruch 8 wobei die neurodegenerativen Krankheiten und neuronalen Schädigungen, durch Ischämie, Trauma oder Massenblutungen ausgelöst werden.

10. Verwendung nach Anspruch 8 wobei die neurodegenerativen krankheiten und neuronalen Schädigungen die Folge eines Schlaganfalls oder eines Schädel-Hirntraumas sind.

11. Verwendung nach Anspruch 8 wobei die neurodegenerativen krankheiten und neuronalen Schädigungen die Folge der Alzheimerschen Krankheit der Parkinsonsche Krankheit oder der Huntington-Krankheit sind.

12. Verwendung von Verbindungen der Formel I bzw. II nach einem der Ansprüche 1 bis 6 zur Herstellung von Arzneimitteln zur Behandlung oder Prophylaxe von Schädigungen durch Ischämien.

13. Verwendung von Verbindungen der Formel I bzw. II nach einem der Ansprüche 1 bis 6 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen.

14. Verwendung von Verbindungen der Formel I bzw. II nach einem der Ansprüche 1 bis 6 zur Herstellung von Arzneimitteln zur Behandlung von Schädigungen der Nieren nach renalen Ischämien und zur Behandlung während und nach Nierentransplantationen.

15. Verwendung von Verbindungen der Formel I bzw. II nach einem der Ansprüche 1 bis 6 zur Herstellung von Arzneimitteln zur Behandlung von Schädigungen des Herzens nach cardialen Ischämien.

16. Verwendung von Verbindungen der Formel I bzw. II nach einem der Ansprüche 1 bis 6 zur Herstellung von Arzneimitteln zur Behandlung von Mikroinfarkten wie zum Beispiel während und nach Herzklappenersatz, Aneurysmenresektionenen und Herztransplantationen.

17. Verwendung von Verbindungen der Formel I bzw. II nach einem der Ansprüche 1 bis 6 zur Herstellung von Arzneimitteln zur Behandlung bei einer Revasculariation kritischer verengter Koronararterien wie zum Beispiel bei PTCA und Bypass-Opera-tionen oder kritisch verengter peripherer Arterien, insbesondere Beinarterien.

18. Verwendung von Verbindungen der Formel I bzw. II nach einem der Ansprüche 1 bis 6 zur Herstellung von Arzneimitteln zur Behandlung des akuten Myocardinfarktes und von Schädigungen während und nach dessen medikamentöser oder mechanischer Lyse.

19. Verwendung von Verbindungen der Formel I bzw. II nach einem der Ansprüche 1 bis 6 zur Herstellung von Arzneimitteln zur Behandlung von Tumoren und deren Metastasierung.

20. Verwendung von Verbindungen der Formel I bzw. II nach einem der Ansprüche 1 bis 6 zur Herstellung von Arzneimitteln zur Behandlung von Sepsis und des septischen Schocks.

21. Verwendung von Verbindungen der Formel I bzw. II nach einem der Ansprüche 1 bis 6 zur Herstellung von Arzneimitteln zur Behandlung von immunologischen Krankheiten wie Entzündungen und rheumatische Erkrankungen, wie zum Beispiel rheumatoide Arthritis.

22. Verwendung von Verbindungen der Formel I bzw. II nach einem der Ansprüche 1 bis 6 zur Herstellung von Arzneimitteln zur Behandlung von Diabetes mellitus.

## Claims

1. Compound of formula I or II 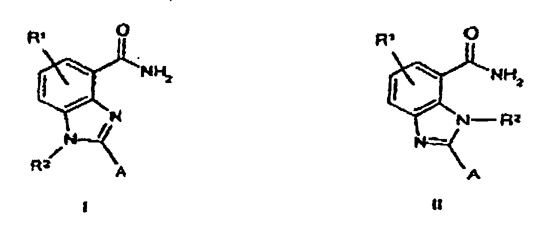 wherein
A is a monocyclic aromatic heterocyclic system, a bicyclic and tricyclic aromatic or partially aromatic heterocyclic system, wherein the ring systems contain not more than 15 carbon atoms and up to 4 hetero atoms selected from the group N, O, S, and rings may additionally carry up to 2 oxo groups, and A may also be substituted by up to three different or identical radicals R³ and additionally a radical R⁴, and
R¹ is hydrogen, chlorine, fluorine, bromine, iodine, branched and unbranched C₁-C₆-alkyl, OH, nitro, CF₃, CN, NR¹¹R¹², NH-CO-R¹³, O-C₁-C₄-alkyl, wherein R¹¹ and R¹², independently of one another, are hydrogen or C₁-C₄-alkyl and R¹³ is hydrogen, C₁-C₄-alkyl, C₁-C₄-alkyl-phenyl or phenyl, and
R² is hydrogen, branched and unbranched C₁-C₆-alkyl, and
R³ is hydrogen, chlorine, bromine, iodine, fluorine, CF₃, OCF₃, nitro, NH₂, CO-R⁸, CO₂-R⁸, SO₂-R⁸, OH, O-C₁-C₄-alkyl, O-C₀-C₄-alkyl-phenyl, a C₁-C₆ chain which may be saturated, unsaturated or partially unsaturated and may also be substituted by a radical R³³, phenyl, wherein the phenyl rings may also be substituted by up to three identical or different radicals R³¹, and pyridyl which may be substituted by up to three radicals R³², and
R³¹ is OH, C₁-C₆-alkyl, O-C₁-C₄-alkyl, chlorine, bromine, iodine, fluorine, CF₃, nitro, NH₂, and
R³² is OH, C₁-C₆-alkyl, O-C₁-C₄-alkyl, chlorine, bromine, iodine, fluorine, CF₃, nitro, NH₂, CN, and
R³³ is CO-NH-R⁸, OH, O-C₁-C₆-alkyl, O-CO-R⁸, and
R⁴ is hydrogen and -(D)ₚ-(E)ₛ-(CH₂)_{q}-B, wherein
D is S, NR⁴³ and O,
E is phenyl, and
s is 0 and 1, and
B is NR⁴¹R⁴² and 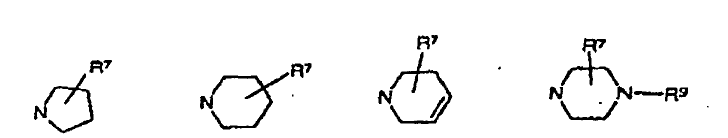 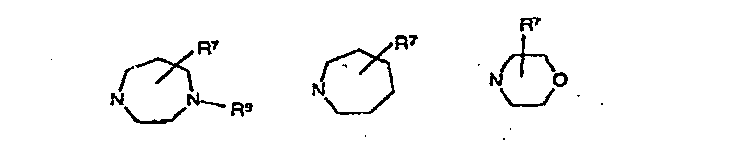
and
p may be 0 and 1, and
q may be 0, 1, 2, 3 or 4, and
R⁴¹ may be hydrogen, C₁-C₆-alkyl, (CH₂)ᵣ-G, and
R⁴² may be hydrogen, C₁-C₆-alkyl, -CO-R⁸, SO₂-R⁸, CO₂-R⁸, -(C=N)-R⁸ and -(C=N)-NHR⁸, and
R⁴¹ and R⁴² may form a phthaloyl radical, and
R⁴³ may be hydrogen and C₁-C₄-alkyl, and
r may be 0, 1, 2, 3, 4, and
G may be phenyl, which may also carry not more than two radicals R, NR¹¹R¹², NH-C₁-C₄-alkyl-phenyl, pyrrolidine, piperidine, 1,2,5,6-tetrahydropyridine, morpholine, homopiperidine, piperazine, which may also be substituted by an alkyl radical C₁-C₆-alkyl, and homopiperazine, which may also be substituted by an alkyl radical C₁-C₆-alkyl, and
R⁷ may be hydrogen, C₁-C₆-alkyl, phenyl, wherein the ring may also be substituted by up to two radicals R⁷¹, and
R⁷¹ may be OH, C₁-C₆-alkyl, O-C₁-C₄-alkyl, chlorine, bromine, iodine, fluorine, CF₃, nitro, NH₂, and
R⁸ may be C₁-C₆-alkyl, CF₃, NR¹¹R¹², phenyl, C₁-C₄-alkyl-phenyl, wherein the ring may also be substituted by up to two radicals R⁸¹, and
R⁸¹ may be OH, C₁-C₆-alkyl, O-C₁-C₄-alkyl, chlorine, bromine, iodine, fluorine, CF₃, nitro, NH₂, and
R⁹ may be hydrogen, CO-R⁸, SO₂-R⁸, CO₂-R⁸, C₁-C₆-alkyl, C₁-C₄-alkyl-phenyl and phenyl, wherein the phenyl rings may also be substituted by up to two radicals R⁹¹, and
R⁹¹ may be OH, C₁-C₆-alkyl, O-C₁-C₄-alkyl, chlorine, bromine, iodine, fluorine, CF₃, nitro, NH₂,
and its tautomeric forms, possible enantiomeric and diastereoisomeric forms, and its prodrugs, selected from the group consisting of phosphates, carbamates of amino acids and esters.

2. Compound of formula I or II according to claim 1, wherein
R¹ may be hydrogen and
R² may be hydrogen and C₁-C₄-alkyl and
D may be NR⁴³ and O and
p may be 0 and 1 and
s may be 0 and
q may be 0, 1 and 2 when p is 0, or q may be 2 and 3 when p is 1, and
R⁴² and R⁴³, independently of one another, may be hydrogen and C₁-C₄-alkyl and
R⁷ may be hydrogen and phenyl and
R⁹ may be hydrogen, C₁-C₄-alkyl and C₀-C₄-alkyl-phenyl.

3. Compounds of formula I or II according to either claim 1 or claim 2, wherein A has the following meaning: indole, benzimidazole, pyrrole, imidazole, furan, thiophene, benzothiophene, benzofuran, pyrazole, thiazole, benzothiazole, phthalimide, indazole, benzotriazole, phthalizine, indoline, isoindoline, pyridine, quinoline, pyrimidine, pyridazine, isoquinoline, quinoxaline, quinazoline, isoxazole, oxazole, imidazopyridine, pyrazine.

4. Compounds of formula I and II according to claim 2, wherein A has the following meaning:
pyridine, thiophene, thiazole, furan, indole, oxazole, pyrazole, pyrrole, benzofuran, imidazole, benzothiophene, isoxazole, pyrazine, pyrimidine, pyridazine, quinoline and the heterocyclic system may be substituted by up to three radicals R³ and a radical R⁴, wherein
R³ is hydrogen, chlorine, bromine, iodine, fluorine, COR⁸, CO₂R⁸, SO₂R⁸, C₁-C₄-alkyl-phenyl, a C₁-C₆ chain which may be saturated, unsaturated and partially unsaturated and may also be substituted by a group O-CO-R⁸, phenyl, wherein the phenyl rings may also be substituted by up to three identical or different radicals R³¹, and pyridyl which may be substituted by up to three identical or different radicals R³², and
R⁴ is hydrogen and (D)ₚ-(E)ₛ-(CH₂)_{q}-B,
and R³ and R⁴ are not simultaneously hydrogen.

5. Compounds of formula I and II according to claim 2, wherein A has the following meaning:
pyridine, pyrazine, pyrimidine, pyridazine, quinoline, thiazole, thiophene, pyrrole and pyrazole and the heterocyclic system may be substituted by a radical R³ and a radical R⁴, wherein
R³ is hydrogen, chlorine, bromine, iodine, fluorine, C₁-C₄-alkyl, and
R⁴ is (D)ₚ-(E)ₛ-(CH₂)_{q}-B.

6. Compounds of formula I and II according to claim 2, wherein A may be pyridine, thiophene and thiazole and the heterocyclic system is substituted by a radical R⁴, wherein R⁴ is (D)ₚ-(E)ₛ-(CH₂)_{q}-B and R³ is hydrogen.

7. Medicament comprising, in addition to conventional carriers and auxiliary substances, compounds according to any one of claims 1 to 3.

8. Use of compounds of formula I or II according to any one of claims 1 to 6 in the preparation of medicaments for the treatment of neurodegenerative diseases and neuronal damage.

9. Use according to claim 8, wherein the neurodegenerative diseases and neuronal damage are caused by ischaemia, trauma or massive bleeding.

10. Use according to claim 8, wherein the neurodegenerative diseases and neuronal damage are the consequence of a stroke or of a craniocerebral trauma.

11. Use according to claim 8, wherein the neurodegenerative diseases and neuronal damage are the consequence of Alzheimer's disease, Parkinson's disease or Huntington's disease.

12. Use of compounds of formula I or II according to any one of claims 1 to 6 in the preparation of medicaments for the treatment or prophylaxis of damage due to ischaemia.

13. Use of compounds of formula I or II according to any one of claims 1 to 6 in the preparation of medicaments for the treatment of epilepsies, especially generalised epileptic seizures, such as, for example, petit mal and tonoclonic seizures, and partial epileptic seizures, such as temporal lobe, and complex partial seizures.

14. Use of compounds of formula I or II according to any one of claims 1 to 6 in the preparation of medicaments for the treatment of damage to the kidneys following renal ischaemia and for treatment during and after kidney transplants.

15. Use of compounds of formula I or II according to any one of claims 1 to 6 in the preparation of medicaments for the treatment of damage to the heart following cardiac ischaemia.

16. Use of compounds of formula I or II according to any one of claims 1 to 6 in the preparation of medicaments for the treatment of microinfarcts such as, for example, during and after heart valve replacement, aneurysm resections and heart transplants.

17. Use of compounds of formula I or II according to any one of claims 1 to 6 in the preparation of medicaments for treatment in the revascularisation of critically narrowed coronary arteries such as, for example, in PTCA and bypass operations or of critically narrowed peripheral arteries, especially leg arteries.

18. Use of compounds of formula I or II according to any one of claims 1 to 6 in the preparation of medicaments for the treatment of acute myocardial infarct and of damage during and after the medical or mechanical lysis thereof.

19. Use of compounds of formula I or II according to any one of claims 1 to 6 in the preparation of medicaments for the treatment of tumours and metastases thereof.

20. Use of compounds of formula I or II according to any one of claims 1 to 6 in the preparation of medicaments for the treatment of sepsis and septic shock.

21. Use of compounds of formula I or II according to any one of claims 1 to 6 in the preparation of medicaments for the treatment of immunological diseases such as inflammations and rheumatic diseases, such as, for example, rheumatoid arthritis.

22. Use of compounds of formula I or II according to any one of claims 1 to 6 in the preparation of medicaments for the treatment of diabetes mellitus.

## Revendications

1. Composé de formule I ou II 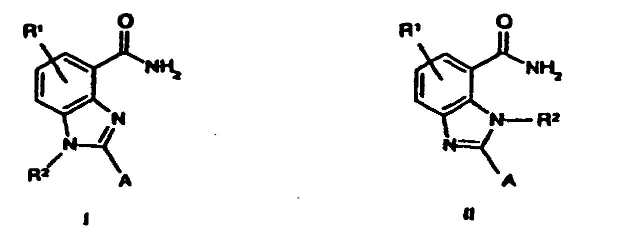 dans lesquelles
A représente un hétérocycle monocyclique aromatique, un système hétérocyclique bicyclique ou tricyclique aromatique ou partiellement aromatique, dans lequel les systèmes cycliques contiennent au plus 15 atomes de carbone et jusqu'à 4 hétéroatomes choisis dans le groupe constitué par N, O, S, et les cycles peuvent en outre porter jusqu'à 2 groupes oxo, et A peut encore être substitué par au plus 3 restes R³ identiques ou différents et en outre par un reste R⁴, et
R¹ représente l'hydrogène, le chlore, le fluor, le brome, l'iode ou un reste alkyle en C₁-C₆ linéaire ou ramifié, OH, nitro, CF₃, CN, NR¹¹R¹², NH-CO-R¹³, O-alkyle en C₁-C₄, où R¹¹ et R¹² représentent, indépendamment l'un de l'autre, l'hydrogène ou un reste alkyle en C₁-C₄ et R¹³ représente l'hydrogène ou un reste alkyle en C₁-C₆, (alkyl en C₁-C₄)-phényle ou phényle, et
R² représente l'hydrogène ou un reste alkyle en C₁-C₆ linéaire ou ramifié, et
R³ représente l'hydrogène, le chlore, le brome, l'iode, le fluor, un reste CF₃, OCF₃, nitro, NH₂, CO-R⁸, CO₂-R⁸, SO₂-R⁸, OH, O-alkyle en C₁-C₄, O-(alkyl en C₀-C₄)-phényle, une chaîne en C₁-C₆ qui peut être saturée, insaturée ou partiellement insaturée et qui peut être encore substituée par un reste R³³, un reste phényle, les cycles phényle pouvant être encore substitués par au plus 3 restes R³¹ identiques ou différents, ou un reste pyridyle pouvant être substitué par au plus 3 restes R³², et
R³¹ représente un reste OH, alkyle en C₁-C₆, O-alkyle en C₁-C₄, chloro, bromo, iodo, fluoro, CF₃, nitro, NH₂, et
R³² représente un reste OH, alkyle en C₁-C₆, O-alkyle en C₁-C₄, chloro, bromo, iodo, fluoro, CF₃, nitro, NH₂, CN et
R³³ représente un reste CO-NH-R⁸, OH, O-alkyle en C₁-C₆, O-CO-R⁸ et
R⁴ représente l'hydrogène ou -(D)ₚ-(E)ₛ-(CH₂)_{q}-B, où
D représente S, NR⁴³ ou O,
E représente un reste phényle et
s est 0 ou 1 et
B représente un reste NR⁴¹R⁴² ou 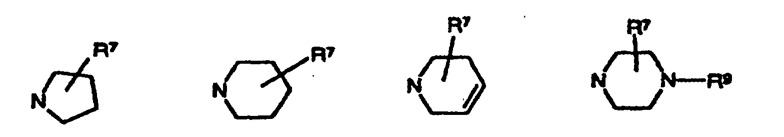 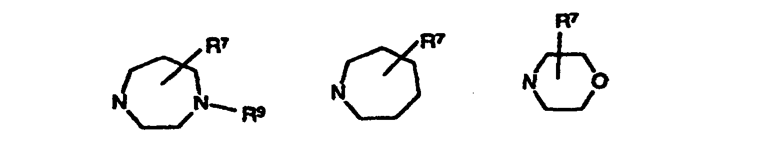 et
p peut être 0 ou 1 et
q peut être 0, 1, 2, 3 ou 4, et
R⁴¹ représente l'hydrogène ou un reste alkyle en C₁-C₆ ou (CH₂)ᵣ-G et
R⁴² représente l'hydrogène ou un reste alkyle en C₁-C₆, -CO-R⁸, SO₂-R⁸, CO₂-R⁸, -(C=N)-R⁸ ou -(C=N)-NHR⁸ et
R⁴¹ et R⁴² peuvent former un reste phtaloyle, et
R⁴³ représente l'hydrogène ou un reste alkyle en C₁-C₄, et
R est 0, 1, 2, 3, 4 et
G représente un reste phényle, qui peut encore porter au plus 2 restes R, un reste NHR¹¹R¹², NH-(alkyl en C₁-C₄)-phényle, pyrrolidine, pipéridine, 1,2,5,6-tétrahydropyridine, morpholine, homopipéridine, un reste pipérazine qui peut encore être substitué par un reste alkyle en C₁-C₆, ou un reste homopipérazine qui peut encore être substitué par un reste alkyle en C₁-C₆, et
R⁷ représente l'hydrogène, un reste alkyle en C₁-C₆, phényle, le cycle pouvant encore être substitué par au plus 2 restes R⁷¹, et
R⁷¹ représente un reste OH, alkyle en C₁-C₆, O-alkyle en C₁-C₄, chloro, bromo, iodo, fluoro, CF₃, nitro, NH₂, et
R⁸ représente un reste alkyle en C₁-C₆, CF₃, NR¹¹R¹², phényle, (alkyl en C₁-C₄)-phényle, le cycle pouvant encore être substitué par au plus 2 restes R⁸¹, et
R⁸¹ représente un reste OH, alkyle en C₁-C₆, O-alkyle en C₁-C₄, chloro, bromo, iodo, fluoro, CF₃, nitro, NH₂, et
R⁹ représente l'hydrogène ou un reste CO-R⁸, SO₂-R⁸, CO₂-R⁸, alkyle en C₁-C₆, (alkyl en C₁-C₄)-phényle ou phényle, les cycles pouvant encore être substitués par au plus 2 restes R⁹¹, et
R⁹¹ représente un reste OH, alkyle en C₁-C₆, O-alkyle en C₁-C₄, chloro, bromo, iodo, fluoro, CF₃, nitro, NH₂,
et ses formes tautomères, ses formes énantiomères et diastéréoisomères possibles, et ses promédicaments, choisis dans le groupe constitué par des phosphates, des carbamates d'aminoacides et des esters.

2. Composé de formule I ou II selon la revendication 1, dans lequel
R¹ représente l'hydrogène et
R² peut être l'hydrogène ou un reste alkyle en C₁-C₄ et
D peut être NR⁴³ ou O et
p peut être 0 ou 1 et
s est 0
q peut être 0, 1 ou 2 lorsque p = 0, ou q peut être 2 ou 3 lorsque p = 1, et
R⁴² et R⁴³, indépendamment l'un de l'autre, peuvent être l'hydrogène ou un reste alkyle en C₁-C₄, et
R⁷ peut être l'hydrogène ou un reste phényle, et
R⁹ peut être l'hydrogène ou un reste alkyle en C₁-C₄ ou (alkyl en C₀-C₄)-phényle.

3. Composés de formule I ou II selon l'une des revendications 1 ou 2, dans lesquels A a la signification suivante: indole, benzimidazole, pyrrole, imidazole, furane, thiophène, benzothiophène, benzofurane, pyrazole, thiazole, benzothiazole, phtalimide, indazole, benzotriazole, phtalizine, indoline, isoindoline, pyridine, quinoléine, pyrimidine, pyridazine, isoquinoléine, quinoxaline, quinazoline, isoxazole, oxazole, imidazopyridine, pyrazine.

4. Composés de formule I et II selon la revendication 2, dans lesquels A a la signification suivante: pyridine, thiophène, thiazole, furane, indole, oxazole, pyrazole, pyrrole, benzofurane, imidazole, benzothiophène, isoxazole, pyrazine, pyrimidine, pyridazine, quinoléine, et l'hétérocycle peut être substitué par au plus 3 restes R³ et un reste R⁴, où
R³ représente l'hydrogène, le chlore, le brome, l'iode, le fluor, un reste COR⁸, CO₂R⁸, SO₂R⁸, (alkyl en C₁-C₄)-phényle, une chaîne en C₁-C₆ qui peut être saturée, insaturée ou partiellement insaturée et qui peut être encore substituée par un groupe O-CO-R⁸, un reste phényle, les cycles phényle pouvant être encore substitués par au plus 3 restes R³¹ identiques ou différents, ou un reste pyridyle pouvant être substitué par au plus 3 restes R³² identiques ou différents, et
R⁴ représente l'hydrogène ou un reste -(D)ₚ-(E)ₛ-(CH₂)_{q}-B,
et R³ et R⁴ ne représentent pas en même temps l'hydrogène.

5. Composés de formule I et II selon la revendication 2, dans lesquels A a la signification suivante: pyridine, pyrazine, pyrimidine, pyridazine, quinoléine, thiazole, thiophène, pyrrole et pyrazole, et l'hétérocycle peut être substitué par un reste R³ et un reste R⁴, où
R³ représente l'hydrogène, le chlore, le brome, l'iode, le fluor, un reste alkyle en C₁-C₄, et
R⁴ représente un reste -(D)ₚ-(E)ₛ-(CH₂)_{q}-B.

6. Composés de formule I et II selon la revendication 2, dans lesquels A peut être un reste pyridine, thiophène ou thiazole et l'hétérocycle est substitué par un reste R⁴, R⁴ étant un reste -(D)ₚ-(E)ₛ-(CH₂)_{q}-B, et R³ représente l'hydrogène.

7. Médicaments contenant, en plus des supports et agents auxiliaires classiques, des composés selon l'une des revendications 1-3.

8. Utilisation de composés de formule I ou II selon l'une des revendications 1 à 6 pour la préparation de médicaments destinés au traitement de maladies neurodégénératives et de lésions neuronales.

9. Utilisation selon la revendication 8, dans laquelle les maladies neurodégénératives et les lésions neuronales sont déclenchées par une ischémie, un traumatisme ou des hémorragies massives.

10. Utilisation selon la revendication 8, dans laquelle les maladies neurodégénératives et les lésions neuronales sont entraînées par une attaque d'apoplexie ou un traumatisme crâniocérébral.

11. Utilisation selon la revendication 8, dans laquelle les maladies neurodégénératives et les lésions neuronales sont entraînées par la maladie d'Alzheimer, la maladie de Parkinson ou la maladie de Huntington.

12. Utilisation de composés de formule I ou II selon l'une des revendications 1 à 6 pour la préparation de médicaments destinés au traitement ou à la prophylaxie de lésions ischémiques.

13. Utilisation de composés de formule I ou II selon l'une des revendications 1 à 6 pour la préparation de médicaments destinés au traitement de l'épilepsie, en particulier des crises d'épilepsie généralisée comme, par exemple, le petit mal et des crises tonico-cloniques et des crises d'épilepsie partielle, comme celles du lobe temporal, et des crises partielles complexes.

14. Utilisation de composés de formule I ou II selon l'une des revendications 1 à 6 pour la préparation de médicaments destinés au traitement de lésions rénales après des ischémies rénales ou au traitement pendant et après des transplantations rénales.

15. Utilisation de composés de formule I ou II selon l'une des revendications 1 à 6 pour la préparation de médicaments destinés au traitement de lésions cardiaques après des ischémies cardiaques.

16. Utilisation de composés de formule I ou II selon l'une des revendications 1 à 6 pour la préparation de médicaments destinés au traitement de microinfarctus comme, par exemple, pendant et après le remplacement d'une valvule cardiaque, des résections d'anévrismes et des greffes cardiaques.

17. Utilisation de composés de formule I ou II selon l'une des revendications 1 à 6 pour la préparation de médicaments destinés au traitement dans une revascularisation d'artères coronaires rétrécies critiques comme, par exemple, dans l'angioplastie coronaire transluminale percutanée (PTCA) ou dans des opérations de pontage, ou d'artères périphériques rétrécies de manière critique, en particulier les artères des jambes.

18. Utilisation de composés de formule I ou II selon l'une des revendications 1 à 6 pour la préparation de médicaments destinés au traitement de l'infarctus aigu du myocarde et de lésions pendant et après sa lyse médicamenteuse ou mécanique.

19. Utilisation de composés de formule I ou II selon l'une des revendications 1 à 6 pour la préparation de médicaments destinés au traitement de tumeurs et de leurs métastases.

20. Utilisation de composés de formule I ou II selon l'une des revendications 1 à 6 pour la préparation de médicaments destinés au traitement de la septicémie et du choc septique.

21. Utilisation de composés de formule I ou II selon l'une des revendications 1 à 6 pour la préparation de médicaments destinés au traitement de maladies immunologiques comme des inflammations et des maladies rhumatismales comme, par exemple, la polyarthrite rhumatoïde.

22. Utilisation de composés de formule I ou II selon l'une des revendications 1 à 6 pour la préparation de médicaments destinés au traitement du diabète sucré.
